# EUROPEAN PATENT SPECIFICATION

(11) **EP 4 064 856 B1**
(45) Date of publication and mention of the grant of the patent: **03.06.2026**
(21) Application number: 20828472.9
(22) Date of filing: 27.11.2020
(51) Int. Cl.: A23L 2/52, A23L 33/21, C08B 37/00, C12C 12/02, C12G 3/05, C12P 19/00, C12P 19/04, C12C 5/02

(54) **HIGH-FIBER, LOW-SUGAR SOLUBLE DIETARY FIBERS, PRODUCTS INCLUDING THEM AND METHODS FOR MAKING AND USING THEM**
FASERREICHE ZUCKERARME LÖSLICHE BALLASTSTOFFE, DIESE ENTHALTENDE PRODUKTE UND VERFAHREN ZU IHRER HERSTELLUNG UND VERWENDUNG
FIBRES ALIMENTAIRES SOLUBLES À HAUTE TENEUR EN FIBRES, À FAIBLE TENEUR EN SUCRE, PRODUITS COMPRENANT CELLES-CI ET PROCÉDÉS DE FABRICATION ET D'UTILISATION DE CELLES-CI

(30) Priority: 28.11.2019 US 201962941778 P; 28.11.2019 US 201962941776 P
(43) Date of publication of application: 05.10.2022
(73) Proprietor: Tate & Lyle Solutions USA LLC, Hoffman Estates, IL 60192 (US)
(72) Inventor: BAHAR, Mark, Hoffman Estates, IL 60192 (US); TOMAR, Juhi, Hoffman Estates, IL 60192 (US); DE SOUZA, Mervyn, Hoffman Estates, IL 60192 (US); EVANS, Annette, Hoffman Estates, IL 60192 (US); FLETCHER, Joshua, Hoffman Estates, IL 60192 (US); CARR, James, Hoffman Estates, IL 60192 (US)
(74) Representative: Marks & Clerk LLP
(86) International application number: PCT/US2020/062522
(87) International publication number: WO 2021/108782

(56) References cited:
- JP-A- 2018 139 554
- US-A1- 2008 175 977
- WESTERLUND E ET AL: "Isolation and chemical characterization of water-soluble mixed-linked @b-glucans and arabinoxylans in oat milling fractions", CARBOHYDRATE POLYMERS, APPLIED SCIENCE PUBLISHERS , LTD BARKING, GB, vol. 20, no. 2, 1 January 1993 (1993-01-01), pages 115 - 123, XP024146969, ISSN: 0144-8617, [retrieved on 19930101], DOI: 10.1016/0144-8617(93)90086-J

## Description

### CROSS-REFERENCE TO RELATED APPLICATIONS

This application claims the benefit of priority of U.S. Provisional Patent Application no. 62/941776, filed November 28, 2019, and of U.S. Provisional Patent Application no. 62/941778, filed November 28, 2019.

### BACKGROUND OF THE DISCLOSURE

### 1. Field of the Disclosure

The present disclosure relates generally to soluble dietary fibers. More particularly, the present disclosure relates to high-fiber, low-sugar soluble dietary fibers, methods for making them, and their use in a variety of food and beverage applications including fermented foods and beverages like beer and sake.

### 2. Technical Background

Soluble dietary fiber is commonly utilized in food and beverage products to provide a variety of desirable characteristics, including nutrition and texture. Among myriad other applications, soluble dietary fibers are of interest for application in fermented beverages to provide a desirable body and mouthfeel. As soluble dietary fiber can be less chemically susceptible to amylolytic enzymes in the mashing process and to fermentation by yeast, it can be used even at early stages in the brewing process to provide desired properties in the final product. This can allow for better control of the properties of the end product. Of course, soluble dietary fiber is desirable for use in a wide variety of other food and beverage products.

Different food and beverage products have different requirements for fiber content, sugar content and rheology. As such, there remains a need for new soluble dietary fibers that have novel combinations of fiber content and sugar content, and with desirable rheological properties for use in different food and beverage applications. JP2018139554A relates to a water-soluble dietary fiber-containing composition and a method for producing the same. Westerlund, Anderson and Aman, Carbohydrate Polymers, 20(2), 1993, 115-123 relates to isolating and chemically characterizing water-soluble mixed-linked β-glucans and arabinoxylans in oat milling fractions. US2008175977A1 relates to a process for making an oligosaccharide composition.

### SUMMARY OF THE DISCLOSURE

One aspect of the disclosure is a soluble dietary fiber having a fiber content of at least 97% as measured by AOAC 2001.03 and a combined DP1 and DP2 content of no more than 3 wt% (e.g., no more than 2 wt%) on a dry solids basis, and a linkage pattern comprising:
30-45% terminally-linked glucopyranosyl residues;
18-30% 6-linked glucopyranosyl residues;
4-12% 4-linked glucopyranosyl residues;
4-13% 3-linked glucopyranosyl residues;
3-8% 2-linked glucopyranosyl residues;
2-10% 4,6-linked glucopyranosyl residues
2-7% 3,6-linked glucopyranosyl residues;
up to 4% 3,4-linked glucopyranosyl residues; and
up to 4% 2,4-linked glucopyranosyl residues.

In one or more embodiments, the soluble dietary fiber has a fiber content of at least 97% as measured by AOAC 2001.03; a combined DP1 + DP2 content of no more than 3 wt% (e.g., no more than 2 wt%) on a dry solids basis; and a weight-average molecular weight in the range of 1000 g/mol to 2500 g/mol, e.g., 1600 g/mol to 2500 g/mol.

Another aspect of the disclosure is a method of making a soluble dietary fiber as otherwise described herein, comprising (i) providing a saccharide feed comprising at least 95% on a dry solids basis of dextrose and/or linear dextrose oligomers; (ii) reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a product composition having a DP3+ of at least 60 wt% on a dry solids basis; and (iii) fractionating the reactor product composition to separate DP1 and DP2 and to provide a high-fiber fraction having a fiber content of at least 97% as measured by AOAC 2001.03 and a combined DP1 and DP2 content of no more than 3 wt% (e.g., no more than 2 wt%) on a dry solids basis as measured by AOAC 2001.03.

In one or more embodiments, the method of making a soluble dietary fiber as otherwise described herein, comprises (i) providing a saccharide feed comprising at least 95% on a dry solids basis of dextrose and/or linear dextrose oligomers; (ii) reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a product composition having a DP3+ of at least 76 wt% on a dry solids basis; and (iii) fractionating the reactor product composition to separate DP1 and DP2 and to provide a high-fiber fraction having a fiber content of at least 97% as measured by AOAC 2001.03 and a combined DP1 and DP2 content of no more than 3 wt% (e.g., no more than 2 wt%) on a dry solids basis as measured by AOAC 2001.03.

In one or more embodiments, the method of making a soluble dietary fiber as otherwise described herein, comprises (i) providing a saccharide feed comprising at least 95% (e.g., at least 97% wt%, at least 98 wt%, or at least 99 wt%) on a dry solids basis of dextrose and/or dextrose oligomers; (ii) reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a product composition having a DP3+ of at least 60 wt% (e.g., at least 76%) on a dry solids basis; and (iii) fractionating the reactor product composition to separate DP1 and DP2 and to provide a high-fiber fraction having a DP1+DP2 value of no more than 3 wt% (e.g., no more than 2 wt%).

Another aspect of the disclosure is a method of improving body in a fermented beverage, the method including providing a soluble dietary fiber as otherwise described herein in the fermented beverage.

Another aspect of the disclosure is a fermented beverage comprising a soluble dietary fiber as otherwise described herein.

Another aspect of the disclosure is a food or beverage comprising a soluble dietary fiber as otherwise described herein, together with allulose.

Another aspect of the disclosure is a method for making a food or beverage product, the method comprising (i) providing a soluble dietary fiber as otherwise described herein; and (ii) combining the soluble dietary fiber with one or more other food or beverage ingredients.

Additional aspects of the disclosure will be evident from the disclosure herein.

### BRIEF DESCRIPTION OF THE DRAWINGS

FIG. 1 is a flowchart depicting a manufacturing process to produce a soluble dietary fiber according to certain embodiments as described herein.
FIG. 2 is a chart comparing saccharide contents of the reactor product composition of Example 1 and the high-fiber product of Example 2.
FIG. 3 is a graph of an experimentally-determined relationship between reactor temperature and DP3+ content of the reactor product composition of a series of example experiments.

### DETAILED DESCRIPTION

There is an ongoing trend in the food and beverage industry to offer low-calorie or no-calorie versions of popular consumables. For example, the low-alcohol (e.g., nonalcoholic) beer market is growing rapidly as consumers seek alternatives that are perceived as healthier or comply with a particular diet. However, the conventional production of low-alcohol beverages, including beer, can suffer from inferior characteristics including a thin mouthfeel and poor head retention. Low-calorie fermented beverages like light beers can similarly have thin mouthfeel and poor head retention; indeed, mouthfeel and head retention are properties that are generally desirable to improve in fermented beverages like beer and sake.

The present inventors have noted that soluble dietary fiber can be used as an ingredient in fermented beverages (e.g., beer and sake, nonalcoholic, low-alcohol or full-alcohol) to improve mouthfeel and head retention. Notably, due to the resistance of fiber to being broken down by a mashing process or to being fermented by yeast in a brewing process, the fiber can be added at any desirable time, including before fermentation (e.g., as part of the grain feed to the mash, during the mash, during lautering), during any fermentation stage, or even post-fermentation and filtering. The soluble dietary fiber can thus provide desired rheological properties to the final beverage product, including an improvement of mouthfeel (i.e., more "body") and an improvement in head retention (i.e., the length of time a foamy "head" of beer persists after pouring).

However, conventional dietary fibers can contain significant amounts of sugars (i.e., monosaccharides and disaccharides), most notably dextrose and dextrose disaccharides like maltose and isomaltose. Some of these sugars can themselves be calorific, and in many cases can be fermented by yeast in a brewing process. Further, in many products, including beer, consumers may not tolerate excessive sweetness imparted by residual sugars not removed by fermentation. As such, the present inventors have noted that a soluble dietary fiber having a high fiber content and low sugar content is desired.

One conventional method of producing soluble dietary fibers is through allowing monosaccharides and oligosaccharides, such as those found in starch hydrolysates like corn syrup, to react in an acid-catalyzed reversion reaction under low-water conditions to provide a product having a relatively low amount of residual sugar, as generally described, for example, in U.S. Patent no. 7,608,436. This approach can produce soluble dietary fiber with an amount of residual sugars and moderate fiber content, which is useful in a number of food and beverage applications. To achieve a higher fiber content, the duration of the polycondensation reaction can be extended in order to consume additional sugar - but the present inventors have noted that this approach can lead to high molecular weights that can provide the product with a rheological profile that is undesirable in uses like beverages.

In view of the observations above, the present inventors have noted a need for an economical soluble dietary fiber ingredient that has a high fiber content and a low sugar content while providing desirable rheological properties. Such a fiber would fulfill an unmet need in a number of applications such as low-calorie and/or low-alcohol beverages such as beer and sake.

Accordingly, one aspect of the disclosure is a soluble dietary fiber having a fiber content of at least 97% as measured by AOAC 2001.03 and a combined DP1 and DP2 content (e.g., a combined content of monosaccharides and disaccharides, such as dextrose and dextrose disaccharides) of no more than 3 wt% (e.g., no more than 2 wt%) on a dry solids basis. This combination of high fiber content and low sugar content can be especially useful in low-calorie applications, applications requiring low sweetness, and applications where significant fermentation of the soluble dietary fiber is undesired.

As described above, a significant attribute of the soluble dietary fiber of the present disclosure is the high fiber content. As used herein, fiber content is measured by AOAC 2001.03 and is recited on a dry solids basis. In certain embodiments as otherwise described herein, the soluble dietary fiber has a fiber content of at least 97%. For example, in certain embodiments as otherwise described herein, the fiber content as measured by AOAC 2001.03 is at least 98%. In certain desirable embodiments as otherwise described herein, the fiber content as measured by AOAC 2001.03 is at least 99%. In various other additional embodiments as otherwise described herein, the fiber content measured by AOAC 2001.03 is in the range of 97% to 110%. For example, in certain embodiments as otherwise described herein, the fiber content as measured by AOAC 2001.03 is in the range of 98% to 110%, e.g., 99% to 110%. In certain embodiments as otherwise described herein, the fiber content as measured by AOAC 2001.03 is in the range of 97% to 108%, e.g., 97% to 106%, or 97% to 103%, or 97% to 100%. In certain embodiments as otherwise described herein, the fiber content as measured by AOAC 2001.03 is in the range of 98% to 108%, e.g., 98% to 106%, or 98% to 103%, or 98% to 100%. In certain embodiments as otherwise described herein, the fiber content as measured by AOAC 2001.03 is in the range of 99% to 108%, e.g., 99% to 106%, or 99% to 103%, or 99% to 100%. The present inventors note that for certain materials as described herein, the AOAC 2001.03 measurement can in some cases provide a fiber content (i.e., on a dry solids basis) in excess of 100%. While the person of ordinary skill in the art will appreciate that values in excess of 100% are in one sense an experimental artifact, it will be understood that real-world fiber measurements according to AOAC 2001.03 may provide values in excess of 100%, with increasing numerical values correlating with increasing fiber. The present inventors note that the high amount of fiber provides significant advantages, e.g., with respect to keto foods and beverages and with respect to use in various alcoholic beverages. Moreover, the high fiber content provides advantages in situations where a particular food or beverage is not amenable to wide variations of composition; the high fiber of these materials means relatively less material can be used in a reformulation to increase fiber or to provide other benefits, e.g., as described herein.

Another significant attribute of the soluble dietary fiber of the present disclosure is the low DP1+DP2 content (i.e., content of monomeric and dimeric material, as commonly understood in the saccharide art). This can be important for applications where low sugar content is desired, or where the sugar content of the food or beverage product comes from other sources. DP1+DP2 content is measured using HPLC, employing an Aminex HPX-87K, 300 x 7.8 mm column, and dextrose as standard, and is provided on a dry solids basis. In certain embodiments as otherwise described herein, the soluble dietary fiber has a DP1+DP2 content of no more than 2.8 wt% on a dry solids basis. For example, in certain embodiments, the DP1+DP2 content is no more than 2.5 wt% on a dry solids basis, e.g., no more than 2.3 wt%. In certain additional embodiments as otherwise described herein, the soluble dietary fiber has a DP1+DP2 content of no more than 2 wt%, e.g., no more than 1.7 wt%. In certain additional embodiments as otherwise described herein, the soluble dietary fiber has a DP1+DP2 content of no more than 1.5 wt%, e.g., no more than 1.2 wt%, or no more than 1.1 wt%. In certain additional embodiments as otherwise described herein, the soluble dietary fiber has a DP1+DP2 content of no more than 1.0 wt%, e.g., no more than 0.8 wt%. In certain additional embodiments as otherwise described herein, the soluble dietary fiber has a DP1+DP2 content of no more than 0.5 wt%. When added before or during fermentation, any remaining fermentable sugars in the soluble dietary fiber can be fermented, e.g., to alcohol.

The individual amounts of DP1 (e.g., monosaccharides) and DP2 materials (e.g., disaccharides) can be important as well as their combined total content. Accordingly, in certain embodiments as otherwise described herein, the soluble dietary fiber has a DP2 content (e.g., a dextrose disaccharide content) of no more than 3 wt% on a dry solids basis, e.g., no more than 2.5 wt%. In certain embodiments as otherwise described herein, the DP2 content (e.g., the dextrose disaccharide content) is no more than 2 wt%, e.g., no more than 1.7 wt%. In certain embodiments as otherwise described herein, the DP2 content (e.g., the dextrose disaccharide content) is no more than 1.5 wt%, e.g., no more than 1.3 wt%. In certain embodiments as otherwise described herein, the DP2 content (e.g., the dextrose disaccharide content) is no more than 1.0 wt%, e.g., no more than 0.75 wt%. In certain embodiments as otherwise described herein, the DP2 content (e.g., the dextrose disaccharide content) is no more than 0.50 wt%.

In certain embodiments as otherwise described herein, the soluble dietary fiber has a DP1 content (e.g., a dextrose content) of no more than 1.0 wt% on a dry solids basis, e.g., no more than 0.75 wt%. In certain embodiments as otherwise described herein, the soluble dietary fiber has a DP1 content (e.g., a dextrose content) of no more than 0.50 wt%, e.g., no more than 0.30 wt%, or no more than 0.20 wt%. In certain embodiments as otherwise described herein, the soluble dietary fiber has a DP1 content (e.g., a dextrose content) of no more than 0.10 wt%, e.g., no more than 0.05 wt%.

Another important attribute of certain embodiments of the present disclosure is a high content of dextrose residues. Notably, the materials of the disclosure can be made to have all or nearly all of the molecular structure (i.e., excluding water) made up of dextrose residues, i.e., either as dextrose monosaccharide or dextrose residues in a DP2+ material. Accordingly, in certain embodiments, the soluble dietary fiber as otherwise described herein has at least 97 wt% dextrose residues on a dry solids basis, e.g., at least 97.5 wt%, at least 98 wt%, or at least 98.5 wt%. That is, at least 97 wt% on a dry solids basis of the material is made up of monomeric dextrose and dextrose residues within a larger molecular structure. In various other additional embodiments as otherwise described herein, the soluble dietary fiber has at least 98.5 wt% dextrose residues, e.g., at least 99 wt%, or at least 99.5 wt%, or at least 99.8 wt%.

Notably, unlike in conventional "polydextrose" products, in certain aspects of the disclosure the soluble dietary fiber does not have substantial amounts of sugar alcohol residues present, either as monomeric sugar alcohols or bound into a larger molecular structure. Desirably, there are substantially no sugar alcohols present. In certain embodiments as otherwise described herein, the soluble dietary fiber has no more than 2 wt% sugar alcohol residues on a dry solids basis, e.g., no more than 1.5 wt% or no more than 1.0 wt%. In certain additional embodiments as otherwise described herein, the soluble dietary fiber has no more than 0.5 wt% sugar alcohol residues on a dry solids basis, e.g., no more than 0.4 wt%, or no more than 0.3 wt%, or no more than 0.2 wt%, or no more than 0.1 wt% sugar alcohol residues. Examples of sugar alcohols include sorbitol, mannitol, xylitol, lactitol, and maltitol.

Notably, certain desirable embodiments of the soluble dietary fiber as otherwise described herein have a desirably low molecular weight. Without intending to be bound by theory, the relatively low molecular weight is believed to advantageously contribute to certain other attributes, such as the rheological properties of the soluble dietary fiber. Notably, the present inventors have provided a soluble dietary fiber that not only has a desirably low molecular weight, but also a high fiber content and a low sugar content as otherwise described herein. In certain especially advantageous embodiments as otherwise described herein, the soluble dietary fiber has a weight-average molecular weight in the range of 1000 g/mol to 2500 g/mol. For example, in various embodiments the weight average molecular weight may be in the range of 1200 g/mol to 2500 g/mol, or 1400 g/mol to 2500 g/mol, or 1500 g/mol to 2500 g/mol, or 1600 g/mol to 2500 g/mol, or 1700 g/mol to 2500 g/mol, or 1800 g/mol to 2500 g/mol, or 1900 g/mol to 2500 g/mol, or 2000 g/mol to 2500 g/mol, or 1200 g/mol to 2400 g/mol, or 1400 g/mol to 2400 g/mol, or 1500 g/mol to 2400 g/mol, or 1600 g/mol to 2400 g/mol, or 1700 g/mol to 2400 g/mol, or 1800 g/mol to 2400 g/mol, or 1900 g/mol to 2400 g/mol, or 1200 g/mol to 2300 g/mol, or 1400 g/mol to 2300 g/mol, or 1500 g/mol to 2300 g/mol, or 1600 g/mol to 2300 g/mol, or 1700 g/mol to 2300 g/mol, or 1800 g/mol to 2300 g/mol, or 1900 g/mol to 2300 g/mol, or 2000 g/mol to 2300 g/mol.

In other embodiments as otherwise described herein, the soluble dietary fiber has a weight-average molecular weight in the range of 1000 g/mol to 3000 g/mol. For example, in various embodiments the weight average molecular weight may be in the range of 1200 g/mol to 3000 g/mol, or 1400 g/mol to 3000 g/mol, or 1500 g/mol to 3000 g/mol, or 1600 g/mol to 3000 g/mol, or 1700 g/mol to 3000 g/mol, or 1800 g/mol to 3000 g/mol, or 1900 g/mol to 3000 g/mol, or 2000 g/mol to 3000 g/mol, or 1200 g/mol to 2750 g/mol, or 1400 g/mol to 2750 g/mol, or 1500 g/mol to 2750 g/mol, or 1600 g/mol to 2750 g/mol, or 1700 g/mol to 2750 g/mol, or 1800 g/mol to 2750 g/mol, or 1900 g/mol to 2750 g/mol, or 2000 g/mol 2750 g/mol.

As used herein, molecular weights of soluble dietary fibers are determined by gel permeation chromatography, using a GPC column set consisting of two Waters Ultrahydrogel 6 micron, 7.8 X 300 mm, 250 Å and 120 Å pore size columns and an Ultrahydrogel DP guard column 6 X 40 mm, and narrow molecular weight distribution pullulans as standards.

As described above, in certain embodiments, the low molecular weight of the soluble dietary fibers of the present disclosure is believed to lead to certain desirable properties. Accordingly, in certain embodiments as otherwise described herein, the soluble dietary fiber has a weight-average molecular weight of no more than 2500 g/mol. For example, in certain embodiments the weight-average molecular weight is no more than 2400 g/mol, or no more than 2300 g/mol. However, in other embodiments, the soluble dietary fibers of the present disclosure have a somewhat higher weight-average molecular weight, e.g., no more than 3000 g/mol, e.g., no more than 2750 g/mol.

In certain embodiments as otherwise described herein, the soluble dietary fiber has a weight-average molecular weight of at least 1200 g/mol. For example, in certain embodiments as otherwise described herein, the molecular weight is at least 1300 g/mol, e.g., at least 1400 g/mol. In various additional embodiments as otherwise described herein, the molecular weight is at least 1500 g/mol, e.g., at least 1550 g/mol, or at least 1600 g/mol, or at least 1700 g/mol. In other embodiments as otherwise described herein, the molecular weight is at least 1800 g/mol, e.g., at least 1900 g/mol, or at least 2000 g/mol.

For example, in certain embodiments as otherwise described herein, the soluble dietary fiber has a weight-average molecular weight in the range of 1600 g/mol to 2500 g/mol e.g., in the range of 1600 g/mol to 2400 g/mol, or in the range of 1900 g/mol to 2300 g/mol.

In certain embodiments as otherwise described herein, the soluble dietary fiber has a number-average molecular weight in the range of 1000 g/mol to 2000 g/mol, e.g., in the range of 1200 g/mol to 1900 g/mol, or in the range of 1400 g/mol to 1800 g/mol. Number-average molecular weights are determined as described above for weight-average molecular weights, processing the data to provide the number-average value as is common in the art.

Moreover, in certain embodiments as otherwise described herein, the soluble dietary fiber has a relatively low polydispersity (i.e., the ratio of weight-average molecular to number-average molecular weight). In certain embodiments as otherwise described herein, the polydispersity of the soluble dietary fiber is no more than 1.8, e.g., no more than 1.7, or no more than 1.6. For example, in certain embodiments, the polydispersity is in the range of 1.1 to 1.8, e.g., 1.2 to 1.7, or 1.25 to 1.6.

The linkage pattern of dextrose residues of the soluble dietary fiber can be important to the properties thereof, including, in various embodiments, digestibility, fermentability and rheology. Accordingly, the soluble dietary fiber has a linkage pattern comprising:
30-45% terminally-linked glucopyranosyl residues;
18-30% 6-linked glucopyranosyl residues;
4-12% 4-linked glucopyranosyl residues;
4-13% 3-linked glucopyranosyl residues;
3-8% 2-linked glucopyranosyl residues;
2-10% 4,6-linked glucopyranosyl residues;
2-7% 3,6-linked glucopyranosyl residues;
up to 4% 3,4-linked glucopyranosyl residues; and
up to 4% 2,4-linked glucopyranosyl residues.

In certain embodiments as otherwise described herein, the soluble dietary fiber has a linkage pattern comprising:
35-43% terminally-linked glucopyranosyl residues;
20-28% 6-linked glucopyranosyl residues;
6-11% 4-linked glucopyranosyl residues;
6-12% 3-linked glucopyranosyl residues;
3-8% 2-linked glucopyranosyl residues;
3-9% 4,6-linked glucopyranosyl residues;
2-7% 3,6-linked glucopyranosyl residues;
up to 3% 3,4-linked glucopyranosyl residues; and
up to 2% 2,4-linked glucopyranosyl residues.

One characteristic parameter for a soluble dietary fiber is the ratio of 6-linked glucopyranosyl residues to 4-linked glucopyranosyl residues. As alpha 1→4 bonds are typically the most susceptible to enzymatic hydrolysis, it can be desirable to have relatively fewer of them. Moreover, different molecular structures can lead to different interactions within the soluble dietary fiber itself as well as with other components in a food or beverage. Different molecular structures will have different molecular shapes, and can provide more or less chain entanglement among the oligosaccharide molecules of the soluble dietary fiber itself, which can provide higher or lower viscosity. And different molecular structures will similarly interact differently with other ingredients in a food or beverage. One example interaction with water; different structures can provide different water activities at the same molecular weight. In certain embodiments as otherwise described herein, the soluble dietary fiber has a ratio of 6-linked glucopyranosyl residues to 4-linked glucopyranosyl residues that is at least 1, e.g., at least 1.5. In certain embodiments, the ratio of 6-linked glucopyranosyl residues to 4-linked glucopyranosyl residues is at least 2, e.g., at least 2.5. In certain embodiments, the ratio of 6-linked glucopyranosyl residues to 4-linked glucopyranosyl residues is at least 3. For example, in certain embodiments, the ratio of 6-linked glucopyranosyl residues to 4-linked glucopyranosyl residues is in the range of 1-4, e.g., in the range of 1.5-4, or 2-4. In certain embodiments, the ratio of 6-linked glucopyranosyl residues to 4-linked glucopyranosyl residues is in the range of 2.5-4, e.g., in the range of 3-4. In certain embodiments, the ratio of 6-linked glucopyranosyl residues to 4-linked glucopyranosyl residues is in the range of 1-3.75, e.g., in the range of 1.5-3.75, or 2-3.75. In certain embodiments, the ratio of 6-linked glucopyranosyl residues to 4-linked glucopyranosyl residues is in the range of 2.5-3.75, e.g., in the range of 3-3.75. In certain embodiments, the ratio of 6-linked glucopyranosyl residues to 4-linked glucopyranosyl residues is in the range of 1-3.5, e.g., in the range of 1.5-3.5, or 2-3.5. In certain embodiments, the ratio of 6-linked glucopyranosyl residues to 4-linked glucopyranosyl residues is in the range of 2.5-3.5, e.g., in the range of 3-3.5.

Linkage patterns are determined using the method of York et al., Methods Enzymol. 116, 3-40 (1985). The method proceeds by permethylating the oligosaccharide, followed by quantitative hydrolysis and acetylation. This results in monomeric species that are acetylated where they were bound to other residues in the oligosaccharide, and methylated everywhere else. The mixture of the monomeric species can be analyzed by gas chromatography to determine relative amounts of different types of linked monomers. All linkages quantified in this disclosure can be determined using this method.

As used herein, a terminal residue is a residue that has only a single linkage to the rest of the oligosaccharide of which is it is a part. A 1,X-linked residue is one that is linked to the rest of the oligosaccharide of which it is a part at through its 1-position and its X-position (i.e., to two other residues). A 1,X,Y-linked residue is one that it is linked to the rest of the oligosaccharide of which it is a part through its 1-position, its X-position, and its Y-position (i.e., to three other residues). As used herein, the term "oligosaccharide" includes disaccharides, trisaccharides, and oligomers of higher degrees of polymerization up to 30. Linkage percentages are provided as the fraction of the total number of terminally-linked residues, di-linked residues and tri-linked residues.

Soluble dietary fibers having the linkage patterns described herein can be advantageously produced using the methods described herein.

Another characteristic parameter for a soluble dietary fiber is the ratio of alpha anomeric protons to beta anomeric protons, i.e., as measured by NMR spectrometry using methods familiar to the person of ordinary skill in the art. In certain embodiments, the soluble dietary fibers described herein have a relatively low ratio of alpha anomeric protons to beta anomeric protons, e.g., no more than 2.5. For example, in certain embodiments, the ratio of alpha anomeric protons to beta anomeric protons is no more than 2, or even no more than 1.8. In certain embodiments, the ratio of alpha anomeric protons to beta anomeric protons is in the range of 1-2.5, e.g., 1-2, or 1-1.8. In certain embodiments, the ratio of alpha anomeric protons to beta anomeric protons is in the range of 1.2-2.5, e.g., 1.2-2, or 1.2-1.8. In certain embodiments, the ratio of alpha anomeric protons to beta anomeric protons is in the range of 1.4-2.5, e.g., 1.4-2, or 1.4-1.8.

In certain embodiments, the soluble dietary fibers described herein can have desirable glass transition temperature. As the person of ordinary skill in the art will appreciate, glass transition temperature will depend on water content. In certain embodiments, the soluble dietary fiber has a glass transition temperature at 70% solids in the range of -20 °C to -50 °C, e.g., -30 °C to -42 °C. Glass transition temperatures are measured by differential scanning calorimetry with a ramp rate of 10 °C per minute. Notably, the soluble fiber products of the disclosure can provide desirably low viscosity. Without intending to be bound by theory, the inventors surmise that this is due to the molecular structure of the soluble dietary fiber, including a relatively low amount of high-molecular weight components. In certain embodiments as otherwise described herein, the soluble dietary fiber has a viscosity at 70% DS and 10 °C of no more than 55000 cP, for example, no more than 50000 cP, or no more than 45000 cP. For example, in certain embodiments, the soluble dietary fiber has a viscosity at 70% DS and 10 °C in the range of 30000-55000 cP, e.g., 30000-50000 cP, or 30000-45000 cP, or 35000-55000 cP, or 35000-50000 cP, or 35000-45000 cP, or 40000-55000 cP, or 40000-50000 cP, or 40000-45000 cP. Viscosities were measured using a stress control (DHR-3) rheometer from TA Instruments, equipped with a lower Peltier plate and an upper parallel plate (40 mm diameter). The upper parallel plate geometry was that of a previous generation of rheometer (AR-2000); in order to make it compatible with the DHR-3 rheometer, a drawdown rod and an adaptor were used. Flow curves at shear rates from 500 s⁻¹ to 0.5 s⁻¹ can be performed at various temperature conditions.

Another aspect of the disclosure is a method of making a soluble dietary fiber as otherwise described herein, comprising (i) providing a saccharide feed comprising at least 95% (e.g., at least 97% wt%, at least 98 wt%, or at least 99 wt%) on a dry solids basis of dextrose and/or dextrose oligomers; (ii) reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a product composition having a DP3+ of at least 60 wt% on a dry solids basis; and (iii) fractionating the reactor product composition to separate the DP1 and DP2 contents and to provide a high-fiber fraction having a fiber content of at least 97% as measured by AOAC 2001.03.

Another aspect of the disclosure is a method of making a soluble dietary fiber as otherwise described herein, comprising (i) providing a saccharide feed comprising at least 95% (e.g., at least 97% wt%, at least 98 wt%, or at least 99 wt%) on a dry solids basis of dextrose and/or dextrose oligomers; (ii) reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a product composition having a DP3+ of at least 60 wt% (e.g., at least 76%) on a dry solids basis; and (iii) fractionating the reactor product composition to separate the DP1 and DP2 content and to provide a high-fiber fraction having a DP1+DP2 value of no more than 3 wt% (e.g., no more than 2 wt%).

It will be understood that the composition of the saccharide feed in the methods as described above can be an important parameter in the production of the soluble dietary fiber as otherwise described herein. As such, in certain desirable embodiments the saccharide feed is a dextrose feed, wherein the dextrose feed includes at least 95 wt% on a dry solids basis of dextrose. For example, in certain embodiments the dextrose feed includes at least 96% wt% dextrose on a dry solids basis, or at least 97 wt%, or at least 98 wt%, or at least 99 wt%, or at least 99.5 wt%, or at least 99.9 wt%.

Additionally, in other embodiments as otherwise described herein, the saccharide feed includes dextrose and dextrose oligomers. For example, in certain desirable embodiments, the saccharide feed includes dextrose and linear dextrose oligomers, i.e., oligomers in which dextrose residues are bonded only by 1,4-alpha linkages. As such, in certain desirable embodiments the saccharide feed is includes at least 95 wt% on a dry solids basis of dextrose and dextrose oligomers (e.g., dextrose and linear dextrose oligomers). For example, in certain embodiments the dextrose feed includes at least 96% wt% dextrose and dextrose oligomers (e.g., dextrose and linear dextrose oligomers) on a dry solids basis, or at least 97 wt%, or at least 98 wt%, or at least 99 wt%, or at least 99.5 wt%, or at least 99.9 wt% dextrose and dextrose oligomers (e.g., dextrose and linear dextrose oligomers) on a dry solids basis. However, in other embodiments, the saccharide feeds includes dextrose but substantially no dextrose oligomers.

In certain embodiments as otherwise described herein, the saccharide feed is a starch hydrolysate having a dextrose equivalence in the range of 25-95. For example, the starch hydrolysate may having a dextrose equivalence in the range of 25-75, e.g., 26-50, 40-70, 60-95, 25-75, or 40-75. These can have varying amounts of dextrose, maltose and higher dextrose oligomers. A variety of starch sources are suitable, e.g., corn, rice, wheat, tapioca and potato.

The saccharide feed is reacted in the presence of water at a total solids concentration of at least 80%. The use of high solids concentration will drive the reaction towards condensation to build to a desired molecular weight (e.g., as described above) and to provide condensation of dextrose residues with one another. Notably, this condensation can provide a variety of different types of bonds, including non-1,4-alpha glucosyl bonds that are not so easily digested by the human digestive system. However, it can be desirable to have some water present to hydrolyze a proportion of any existing 1,4-alpha bonds in the feed (e.g., in linear dextrose oligomers), and in any event the production process can tolerate a degree of water present. The person of ordinary skill in the art will select a solids content in conjunction with other process conditions to provide a desired soluble dietary fiber. For example, in certain embodiments as otherwise described herein, the reaction is performed at a total solids concentration of at least 85%, or even at least 90%. In various embodiments as otherwise described herein, the reaction is performed at a total solids concentration the range of 80 wt% to 99 wt%, e.g., 85-99 wt%, or 90-99 wt%, or 93-99 wt%, or 80-98 wt%, or 85-98 wt%, or 90-98 wt%, or 92-98 wt%, or 93-98 wt%, or 80-96 wt%, or 85-96 wt%, or 90-96 wt%, or 93-96 wt%.

Of course, a saccharide feed can be provided at a relatively lower solids content (e.g., a pumpable syrup at 60-70%), then concentrated under the reaction conditions to the ultimate desired solids content for the reaction. The reaction can be performed while allowing escape of water (e.g., passively by venting or actively under vacuum), to not only concentrate a lower solids feed but also to drive the condensation by removal of water. It can be desirable to add portions of water to maintain the solids content at a desirable level (e.g., 93-98 wt% or any other amount described above) as water is removed from the system.

As condensation will produce water, the reaction can be performed while allowing water to escape the system, e.g., passively through venting the system, or actively using vacuum pumping.

Notably, the reaction is performed in the substantial absence of sugar alcohols, as is consistent with the fact that the soluble dietary fibers of the disclosure are not "polydextroses." As used herein, a "substantial absence of sugar alcohols" means no more than 0.5 wt% of the feed. Desirably, the reaction is performed with no more than trace amounts of sugar alcohol present.

The reaction is performed at a temperature of at least 120 °C. The person of ordinary skill in the art will select a solids content in conjunction with other process conditions to provide a desired soluble dietary fiber. For example, in certain embodiments as otherwise described herein, the reaction is performed at a temperature of at least 130 °C, at least 140°C, or even at least 149 °C. In various embodiments as otherwise described herein, the saccharide feed is reacted at a temperature of no more than 350°C, e.g., no more than 340 °C, or no more than 330 °C, or no more than 325 °C. In certain embodiments as otherwise described herein, the saccharide feed is reacted as a temperature of no more than 320 °C, e.g., no more than 310 °C, or no more than 300 °C, or no more than 290 °C, or no more than 280°C. In certain embodiments as otherwise described herein, the saccharide feed is reacted as a temperature of no more than 270 °C, or no more than 260 °C, or no more than 250 °C, or no more than 245 °C, or no more than 240 °C, or no more than 235 °C, or no more than 230 °C, or no more than 225 °C, or no more than 220°C. In various embodiments as otherwise described herein, the reaction is performed at a temperature in the range of 120 °C to 190 °C, e.g., 120-180 °C, or 120-170 °C, or 130-190 °C, or 130-180 °C, or 130-170 °C, or 140-190 °C, or 140-180 °C, or 140-170 °C, or 140-190 °C, or 140-180 °C, or 140-170 °C. However, in other embodiments, the reaction is performed at a temperature in the range of 200 °C to 300 °C, e.g., in the range of 220 °C to 300 °C, or in the range of 240 °C to 300 °C, or in the range of 260 °C to 300 °C, or in the range of 200 °C to 280 °C, or in the range of 220 °C to 280 °C, or in the range of 240 °C to 280°C, or in the range of 260 °C to 280°C, or in the range of 200 °C to 260 °C, or in the range of 220 °C to 260°C, or in the range of 240 °C to 260°C, or in the range of 200 °C to 240°C, or in the range of 220 °C to 240°C, or in the range of 200 °C to 220°C. And in other embodiments, the reaction is performed at a temperature in the range of 250 °C to 350 °C, e.g., in the range of 250 °C to 325 °C, or in the range of 275 °C to 350°C, or in the range of 275 °C to 325 °C, or in the range of 300 °C to 350°C, or in the range of 325 °C to 350 °C.

A variety of acid catalysts are known to catalyze the formation and hydrolysis of glucosyl bonds. For example, in certain embodiments, the at least one acid catalyst is selected from hydrochloric acid, phosphoric acid and sulfuric acid. In some embodiments, a combination of acids is used, for example a combination of hydrochloric acid and phosphoric acid. Of course, other acid catalysts may also be suitable, e.g., citric acid, acetic acid, malic acid. However, in certain desirable embodiments, no carboxylic acid catalyst is used. In certain embodiments, at least part of the acid catalyst is present from earlier processing (e.g., from the formation of a starch hydrolysate used as feed). The person of ordinary skill in the art will select an amount of acid suitable to provide a desired reaction rate in view of other reaction conditions. For example, in certain embodiments, sufficient acid is present to provide a reaction mixture pH of no more than 4, e.g., no more than 3 or no more than 2.5, such as in the range of 1.0-2.5.

The reaction time will vary depending on reaction conditions, as the person of ordinary skill in the art will appreciate. A wide variety of times can be used. However, in certain embodiments, the reaction time (i.e., time under the recited temperature, acid and solids content conditions) is in the range of 0.1-60 minutes, e.g., 0.1-30 minutes, or 0.1-15 minutes, or 0.1-10 minutes, or 0.5-60 minutes, or 0.5-30 minutes, or 0.5-15 minutes, or 0.5-10 minutes, or 1-60 minutes, or 1-30 minutes, or 1-15 minutes, or 1-10 minutes.

The reaction can be performed in any convenient system, e.g., in a batch reactor, or in a continuous reactor (e.g., a pipe) with continuous flow.

As described above, the saccharide feed is reacted to form a product composition with a DP3+ content of at least 60 wt% on a dry solids basis. In certain embodiments as otherwise described herein, the reactor product composition has a DP3+ of at least 61 wt% on a dry solids basis, e.g., at least 62 wt%, at least 63 wt%, or at least 64 wt%. In various other embodiments, the reactor product composition has a DP3+ content in the range of 60-74 wt%, or in the range of 61-74 wt%, or in the range of 62-74 wt%, or in the range of 60-72 wt%, or in the range of 62-72 wt%, or in the range of 60-70 wt%, or in the range of 62-70 wt%, or in the range of 60-68 wt%, or in the range of 62-68 wt%.

In certain other embodiments as otherwise described herein, the reaction conditions can be modified such that the reactor product composition has a DP3+ of at least 76 wt% on a dry solids basis. For example, the DP3+ content of the reactor product composition may be at least 77 wt%, e.g., at least 78 wt%, or at least 79 wt%, or at least 80 wt%. In various other embodiments, the reactor product composition has a DP3+ content in the range of 76-86 wt% on a dry solids basis, e.g., 78-86 wt%, or 80-86 wt%, or 82-86 wt%, or 76-84 wt%, or 78-84 wt%, or 80-84 wt%, or 82-84 wt%, or 78-82 wt%, or 80-82 wt%. And in various other embodiments, the reactor product composition has a DP3+ content in the range of 82-94 wt% on a dry solids basis, e.g., 82-92 wt%, or 82-90 wt%, or 82-88 wt%, or 84-94 wt%, or 84-92 wt%, or 84-90 wt%, or 84-88 wt%, or 86-94 wt%, or 86-92 wt%, or 86-90 wt%, or 88-94 wt%, or 88-92 wt%.

The method as described above also produced a product composition having a defined sugar content, as measured by the DP1 and DP2 content. Accordingly, in certain embodiments as otherwise described herein, the combined DP1 and DP2 content is between 10 wt% and 22 wt% on a dry solids basis. For example, the combined DP1 and DP2 content may be between 12 wt% and 20 wt%, or between 15 wt% and 20 wt%, or between 10 wt% and 15 wt%, or between 15 wt% and 18 wt%.

After the formation of the reactor product composition as described above, the method of making the soluble dietary fiber includes a fractionation step. The fractionation step serves to remove DP1 and DP2 sugars from the soluble dietary fiber. The fractionation step may also be useful in modifying the weight-average molecular weight of the final product. In certain embodiments as otherwise described herein, fractionating the reactor product composition removes at least 80% of the combined DP1 and DP2 content of the reactor product composition. In various other embodiments as otherwise described herein, fractionating the reactor product composition removes at least 85% of the combined DP1 and DP2 content of the reactor product composition, e.g., removes at least 90% of the combined DP1 and DP2 content of the reactor product composition, or removes at least 92% of the combined DP1 and DP2 content of the reactor product composition, or removes at least 94% of the combined DP1 and DP2 content of the reactor product composition. The level of sugar removal may be determined by one of skill in the art, based on the present disclosure, to target a particular product composition, maximize yield, or improve manufacturing properties.

Fractionation can be performed, for example, to selectively remove lower molecular weight components (e.g., DP1, or DP1+DP2, or DP1-DP3) as compared to higher molecular weight components, providing a high-fiber fraction. As described above, in certain aspects of the disclosure, the high-fiber fraction may have a fiber content of at least 97% as measured by AOAC 2001.03. For example, in certain embodiments as otherwise described herein, the fiber content as measured by AOAC 2001.03 of the high-fiber fraction is at least 98%. In certain desirable embodiments as otherwise described herein, the fiber content of the high-fiber fraction as measured by AOAC 2001.03 is at least 99%. In various other additional embodiments as otherwise described herein, the fiber content of the high-fiber fraction as measured by AOAC 2001.03 is in the range of 97% to 110%. For example, in certain embodiments as otherwise described herein, the fiber content of the high-fiber fraction as measured by AOAC 2001.03 is in the range of 98% to 110%, e.g., 99% to 110%. In certain embodiments as otherwise described herein, the fiber content of the high-fiber fraction as measured by AOAC 2001.03 is in the range of 97% to 108%, e.g., 97% to 106%, or 97% to 103%, or 97% to 100%. In certain embodiments as otherwise described herein, the fiber content of the high-fiber fraction as measured by AOAC 2001.03 is in the range of 98% to 108%, e.g., 98% to 106%, or 98% to 103%, or 98% to 100%. In certain embodiments as otherwise described herein, the fiber content of the high-fiber fraction as measured by AOAC 2001.03 is in the range of 99% to 108%, e.g., 99% to 106%, or 99% to 103%, or 99% to 100%. The high-fiber fraction can itself be used as a soluble dietary fiber of the disclosure, or, as the person of ordinary skill in the art, can be further processed and/or polished to provide a soluble dietary fiber of the disclosure.

The fractionation can be performed to provide the high-fiber fraction with a low degree of DP1+DP2, a low content of DP2, and/or a low content of DP1, in the amounts described above with respect to the soluble dietary fibers of the disclosure. That is, the fractionation can provide a high-fiber fraction suitable for use as a soluble dietary fiber of the disclosure, e.g., as-is, or with further purification or polishing that does not substantially change the DP1/DP2 content of the material.

It will be understood that a multitude of fractionation technologies exist that may be useful for fractionating the reactor product composition, including membrane filtration and various chromatographic techniques. In certain embodiments as otherwise describe herein, the fractionation is performed by chromatography. In various other embodiments as otherwise described herein, the fractionation is performed by sequential simulated moving bed chromatography. Fractionation techniques useful for the processes described herein are described, e.g., in U.S. Patent Application Publication no. 2012/0034366.

Further processing by enzymes can also be used, e.g., before or after any fractionation steps. However, in certain embodiments no processing by enzymes is performed at any point during the reaction or purification sequence.

The person of ordinary skill in the art will appreciate that conventional methodologies can be used for further polishing and purifying the product, e.g., decolorization and ion exchange.

Soluble dietary fibers are often used to modify the texture, thickness, mouthfeel, body or other physical aspects of a food or beverage product. As described above, the soluble dietary fibers of the present disclosure are well-suited for use in fermented beverages, e.g., beer. Accordingly, another aspect of the disclosure is a method of improving body in a fermented beverage, the method including providing a soluble dietary fiber as otherwise described herein in the fermented beverage. In certain embodiments as otherwise described herein, providing the soluble dietary fiber in the fermented beverage includes providing a fermented beverage and combining the soluble dietary fiber in the fermented beverage. As such, another aspect of the disclosure is a fermented beverage comprising a soluble dietary fiber as otherwise described herein.

One advantage of the soluble dietary fiber of the present invention is its low sugar content and relative resistance for enzymatic breakdown, including during fermentation. As such, soluble dietary fiber as otherwise described herein may be added prior to a fermentation step. Accordingly, in certain embodiments as otherwise described herein, providing the soluble dietary fiber in the fermented beverage includes providing a fermentable wort, combining the soluble dietary fiber with the fermentable wort, and fermenting the fermentable wort including the soluble dietary fiber to provide the fermented beverage. Alternatively, in various other embodiments, providing the soluble dietary fiber in the fermented beverage includes providing a mash by combining grains and water, including the soluble dietary fiber in the mash, collecting a fermentable wort including the soluble dietary fiber from the mash, and fermenting the fermentable wort to provide the fermented beverage.

It will be understood that the soluble dietary fiber of the present invention can be provided in a number of fermented beverages. In certain embodiments as otherwise described herein, the fermented beverage is a beer (e.g., an ale or a lager). In various other embodiments, the fermented beverage is a cider, a mead, a wine, a rice wine, a sake, a kombucha or a sauerkraut juice. Suitable fermented beverages can be filtered or unfiltered, and can be pasteurized as well as unpasteurized. Notably, the soluble dietary fibers described herein can be especially useful in unfiltered and unpasteurized beverages, in that they can be resistant to fermentation by any residual yeast present.

In certain embodiments, the fermented beverage contains alcohol. For example, the fermented beverage may contain ethanol. In certain embodiments as otherwise described herein, the fermented beverage contains no more than 20 vol% ethanol, or no more than 15 vol% ethanol, or no more than 8 vol% ethanol. In various other embodiments as otherwise described herein, the fermented beverage has been treated to remove alcohol. Various methods to remove alcohol are known in the art, including evaporative processes (e.g., vacuum distillation) and/or reverse osmosis. Accordingly, in certain embodiments as otherwise described herein, the fermented beverage contains no more than 1.2 vol% ethanol (e.g., ethanol), or no more than 1.0 vol% ethanol, or no more than 0.75 vol% ethanol, or no more than 0.5 vol% ethanol, or no more than 0.2 vol% ethanol. In various embodiments as otherwise described herein, the fermented beverage contains between 0.10 vol% and 1.2 vol% ethanol. For example, the fermented beverage may contain between 0.2 vol% and 1.20 vol% ethanol, or between 0.5 vol% and 1.2 vol% ethanol, or between 0.5 vol% and 1.0 vol% ethanol. In certain other embodiments as otherwise described herein, the fermented beverage contains essentially no alcohol, e.g., no more than 0.2 vol% ethanol, or no more than 0.10 vol% ethanol, or no more than 0.05 vol% ethanol.

Another aspect of the disclosure is a method for making a food or beverage product, the method comprising (i) providing a soluble dietary fiber as otherwise described herein; and (ii) combining the soluble dietary fiber with one or more other food or beverage ingredients. The food or beverage product comprising the soluble dietary fiber as otherwise described herein may also be fermented or cultured.

A variety of food and beverage products can benefit from the addition of the soluble dietary fiber of the present invention. In certain embodiments, the food or beverage product may be a spirit, a liqueur or a spirit alternative (e.g., low-alcohol or low-calorie spirit). In certain other embodiments the food or beverage product is a cocktail or mixed drink, e.g., a margarita, old-fashioned, mulled wine or egg nog. The soluble dietary fiber may also be included in powdered or concentrated mixes wherein the consumer creates the final product. Accordingly, in further embodiments as otherwise described herein, the food or beverage product is a mix such as a cocktail mix (e.g., margarita mix) or hot chocolate mix.

The soluble dietary fibers described herein can be useful for products with low/no digestible carbohydrates due to their very high levels of dietary fiber and low levels of digestible sugars. Such low/no digestible carbohydrate products are amenable for use in entering and maintaining ketosis (or "Keto"), a metabolic state useful for weight loss and for certain medical conditions such as epilepsy, in which most of the body's energy supply comes from ketone bodies in the blood, in contrast to a state of glycolysis in which blood glucose provides most of the energy. In glycolysis, higher levels of insulin promote storage of body fat and block release of fat from adipose tissues. In contrast, while in ketosis fat reserves are readily released and consumed. Ketosis is generally characterized by serum concentrations of ketone bodies over 0.5 mM, with low and stable levels of insulin and blood glucose, both of which can be promoted when consuming high fiber and low/no carbohydrate products made with the soluble dietary fibers described herein. One disadvantage of conventional Keto diets is the lack of carbohydrates believed to be essential in a regular diet. Fortification with the soluble dietary fibers described herein can allow for the addition of fiber desirable for digestive health and microbiome support to balance out the protein consumption without adding a significant amount of digestible sugars. Thus, the soluble dietary fibers described herein can be used in keto food and beverage products, e.g., those having no more than 10% of their calories from carbohydrate.

The soluble dietary fibers described herein can be useful for sports nutrition, due to their very high levels of dietary fiber and low levels of digestible sugars. Products in the sports nutrition category require targeted amounts of nutrients per serving, low/no sugars, low digestible carbohydrates etc. Fortification with the soluble dietary fibers described herein can allow for the addition of fiber desirable for digestive health and microbiome support without adding a significant amount of digestible sugars.

In various other embodiments, the food or beverage product is a dairy product. For example, the food or beverage product is a dairy drink, dairy drink with added fruit or cereal grains, dairy-based smoothie, yogurt, kefir, drinkable yogurt, long shelf life yogurt, dairy-based meal replacement drink, dairy-based drink mix, quark, ice cream or egg nog. In various other embodiments, the food or beverage product is a dairy alternative, e.g., nut milk, oat milk, dairy-free beverage mix, cereal or grain drink, almond milk, rice milk, cashew milk, soy milk, hemp milk or coconut milk. The soluble dietary fiber as otherwise described herein can provide fiber fortification and potentially allow for the provision of low/no/reduced sugar beverages, while providing enhanced mouthfeel in such beverages, especially in the case of nut and seed milks which often can suffer from a thin mouthfeel. Enhanced digestive health, weight management, and increase in satiety can also be provided through the use of the soluble dietary fibers described herein.

In certain embodiments as otherwise described here, the food or beverage product is a juice or fruit/vegetable drink (e.g., fruit juice, concentrated juice mix, vegetable juice, vegetable juice mix, blended juice, fruit or vegetable puree or coulis). In further embodiments, the food or beverage product is a water, e.g., flavored water, unflavored water, sparkling water, carbonated water, flavored water mix or sparkling water mix.

The soluble dietary fibers described herein can also be used in tea and coffee drinks. Here, too, the soluble dietary fibers described herein can provide enhanced mouthfeel, together with low/no/reduced sugar and enhanced digestive health. Suitable products include, for example, tea or coffee drink mix, textured tea or textured coffee, a tea or coffee for enhanced digestive health, cold brew coffee, prepackaged coffee or tea drinks.

In various other embodiments as otherwise described herein, the food or beverage product is a coarse grain food or beverage product, e.g., beverage made with coarse grains, coarse grain drink mix, coarse grain drink combined with juice or dairy drink or coffee drink or tea drink or fermented drink. The food or beverage product can also be a slimming beverage or meal replacement beverage.

In certain embodiments, the food or beverage product is a bar (e.g., a snack bar), for example, a meal replacement bar, a nutrition bar, a granola bar, a cereal bar, a grain bar, a protein bar or a nut bar. In various other embodiments, the food or beverage product is a granola, a muesli, a topping, a coating, a baked good (e.g., cookie, a biscuit, a bread, a pastry, a pizza crust, a flatbread), a bar (e.g., snack bar, cereal bar, granola bar, energy bar), a meat alternative, a filling (e.g., a fruit filling or a crème filling), a fruit snack such as a fruit leather, a pasta, a sweetener, a frozen dessert, a dairy product (e.g., a yogurt, a quark, an ice cream), a dairy alternative product (e.g. yoghurt alternative), a glaze, a frosting, a syrup, a pet food, a medical food, a flavoring or a dry blend.

A soluble dietary fiber as described herein can be used in food or beverage products in combination with bulking agents, such as sugar alcohols or maltodextrins, to reduce caloric content and/or to enhance nutritional profile of the product. A soluble dietary fiber as described herein can also be used as a partial replacement for fat in food or beverage products. Moreover, a soluble dietary fiber can be included in a food or beverage product in combination with a variety of sweeteners. Sweeteners such as sucrose, fructose, corn syrup. Moreover, so-called high-intensity sweeteners such as steviol glycosides, aspartame, sucralose, saccharin, neotame, advantame, acesulfame potassium, monkfruit-based sweeteners can also be used. In certain embodiments as otherwise described herein, the combination of soluble dietary fiber and sweetener can provide for an increased perception of sweetness as compared to the sweetener alone, despite the soluble dietary fiber itself having relatively low sugar content.

In certain embodiments, the soluble dietary fiber is provided in combination with allulose (i.e., D-allulose, also known as D-psicose). Allulose can provide a number of advantages in food and beverage compositions. It provides sweetness without significant caloric content. Moreover, it does not hydrolyze under reduced pH conditions and as such can provide consistent viscosity, taste, sweetness and mouthfeel. Notably, allulose can help to mask certain flavors, e.g., alcoholic harshness in an alcoholic beverage. And allulose can provide flavor modification even at levels beneath the sweetness threshold. In combination with the soluble dietary fibers described herein, allulose can improve body and mouthfeel, in some cases synergistically. When allulose is used at low levels, the soluble dietary fiber can provide body and mouthfeel.

A soluble dietary fiber as described herein can be used in food or beverage products as a tenderizer or texturizer, to increase crispness or snap, to improve eye appeal, and/or to improve the rheology of dough, batter, or other food compositions. A soluble dietary fiber as described herein can also be used in food products as a humectant, to increase product shelf life, and/or to produce a softer, moister texture. It can also be used in food products to reduce water activity or to immobilize and manage water. Additional uses of the oligomer composition as described herein include: to replace egg wash and/or to enhance the surface sheen of a food product, to alter flour starch gelatinization temperature, and to modify the texture of the product.

At least in some embodiments of the invention, a soluble dietary fiber as described herein has one or more of the following advantages: high solubility, which makes it relatively easy to incorporate into food compositions, such as batters and doughs; stability under elevated temperatures and/or acidic pH (some other soluble fibers, such as inulin, are not as stable), lower sweetness, clean flavor, and clear color. The properties of a soluble dietary fiber as described herein can allow food or beverage products in which it is used to have a so-called "clean label."

A soluble dietary fiber as described herein can be used in a variety of types of food or beverage products. One type of food product in which a soluble dietary fiber as described herein can be very useful is bakery products (i.e., baked foods), such as cakes, cheesecakes, baked mousses, brownies, cookies, cookie crisps, muffins, breads, and sweet doughs. Conventional bakery products can be relatively high in sugar and high in total carbohydrates. The use of a soluble dietary fiber as described herein as an ingredient in bakery products can help lower the sugar and carbohydrate levels, as well as reduce the total calories, while increasing the fiber content of the bakery product.

There are two main categories of bakery products: yeast-raised and chemically-leavened. In yeast-raised products, like donuts, sweet doughs, and breads, a soluble dietary fiber as described herein can be used to replace sugars, but a small amount of sugar may still be desired due to the need for a fermentation substrate for the yeast or for crust browning. A soluble dietary fiber as described herein in solid form could be added in a manner similar to nutritive dry sweeteners, with other dry ingredients, and would require no special handling. A soluble dietary fiber as described herein can be added with other liquids as a direct replacement for syrups or liquid sweeteners. The dough would then be processed under conditions commonly used in the baking industry including being mixed, fermented, divided, formed or extruded into loaves or shapes, proofed, and baked or fried. The product can be baked or fried using conditions similar to traditional products. Breads are commonly baked at temperatures ranging from 420 °F. to 520 °F. for 20 to 23 minutes and doughnuts can be fried at temperatures ranging from 400-415 °F., although other temperatures and times could also be used. High intensity sweeteners can be added to doughs as required to obtain optimum sweetness and flavor profile.

Chemically leavened products typically have more sugar and may contain have a higher level of a soluble dietary fiber as described herein. A finished cookie can contain 30% sugar, which could be replaced, entirely or partially, with a soluble dietary fiber as described herein. These products could have a pH of 4-9.5, for example. The moisture content can be between 2-40%, for example.

A soluble dietary fiber as described herein is readily incorporated and may be added to the fat at the beginning of mixing during a creaming step or in any method similar to the syrup or dry sweetener that it is being used to replace. The product would be mixed and then formed, for example by being sheeted, rotary cut, wire cut, or through another forming process. The products would then be baked under typical baking conditions, for example at 200-450 °F.

A soluble dietary fiber as described herein can also be used to form sugar glasses in the amorphous state, to adhere particles to baked goods, and/or used to form a film or coating which enhances the appearance of a baked good. A soluble dietary fiber as described herein in solid form, like other amorphous sugars, form glasses with heating and subsequent cooling to a temperature below their glass transition temperature.

Another type of food or beverage product in which a soluble dietary fiber as described herein can be used is breakfast cereal. For example, a soluble dietary fiber as described herein could be used to replace all or part of the sugar in extruded cereal pieces and/or in the coating on the outside of those pieces. The coating is typically 30-60% of the total weight of the finished cereal piece. A soluble dietary fiber as described herein can be applied in a spray or drizzled on, for example. The formula for the coating can be as simple as a 75% solution of a soluble dietary fiber as described herein. A soluble dietary fiber as described herein could also be blended with sugar at various percentages, or with other sweeteners or polyols. The extra moisture could then be evaporated in a low heat oven. In an extruded piece, a soluble dietary fiber as described herein in solid form could be added directly with the dry ingredients, or a soluble dietary fiber as described herein in syrup form could be metered into the extruder with water or separately. A small amount of water could be added in the extruder, and then it could pass through various zones ranging from 100 °F. to 300 °F. Optionally, other sources of fiber such as resistant starch can be used in the extruded piece. Using a soluble dietary fiber as described herein would create a different texture than other fiber sources. Using it alone or in combination with other fibers may alter the texture to create product diversity.

Another type of food product in which a soluble dietary fiber as described herein can be used is confections. Examples of confections in which it can be used include hard candies, fondants, nougats and marshmallows, gelatin jelly candies or gummies, jellies, wine gums, chocolate, liquor chocolates, chocolates and confectionary items with liquor fillings, confectionery coating, licorice, chewing gum, caramels and toffees, chews, mints, tableted confections, hard-panned and soft panned products, and fruit snacks. In fruit snacks, a soluble dietary fiber as described herein could be used in combination with fruit juice. The fruit juice would provide the majority of the sweetness, and the soluble dietary fiber as described herein would reduce the total sugar content and add fiber. The syrup can be added to the initial candy slurry and heated to the finished solids content. The slurry could be heated from 200-305° F. to achieve the finished solids content. Acid could be added before or after heating to give a finished pH of 2-7. A soluble dietary fiber as described herein could be used as a replacement for 0-100% of the sugar and 1-100% of the corn syrup or other sweeteners (e.g., tapioca syrup, pea syrup) present.

Another type of food product in which a soluble dietary fiber as described herein can be used is spreads, such as nut-based spreads. Examples include highly sweetened spreads such as sweetened hazelnut spreads (e.g., NUTELLA); and nut butters such as peanut butter, almond butter and cashew butter, which are often sweetened (albeit to a lower degree than NUTELLA). Of course, soluble dietary fiber can be used as described herein even in unsweetened nut butters. The use of soluble dietary fiber can provide enhanced sweetness and/or flavor as described herein, and can also provide desirable texture to the spread.

Another type of food product in which a soluble dietary fiber as described herein can be used is jams and jellies. Jams and jellies are made from fruit. A jam contains fruit pieces, while jelly is made from fruit juice. A soluble dietary fiber as described herein can be used in place of sugar or other sweeteners as follows: Weigh fruit and juice into a tank. Premix sugar, resistant corn syrup and pectin. Add the dry composition to the liquid and cook to a temperature of 214-220 °F. Hot fill into jars and retort for 5-30 minutes.

Another type of food product in which a soluble dietary fiber as described herein can be used is high solids fillings. Examples of high solids fillings in which it can be used include fillings in snack bars, toaster pastries, donuts, and cookies. The high solids filling could be an acid/fruit filling or a savory filling, for example. It could be added to products that would be consumed as is, or products that would undergo further processing, by a food processor (additional baking) or by a consumer (bake stable filling). In some embodiments, the high solids fillings would have a solids concentration between 67-90%. The solids could be entirely replaced with a soluble dietary fiber as described herein, or it could be used for a partial replacement of the other sweetener solids present (e.g., replacement of current solids from 5-100%). Typically fruit fillings would have a pH of 2-6, while savory fillings would be between 4-8 pH. Fillings could be prepared cold, or heated at up to 250 °F. to evaporate to the desired finished solids content.

Another type of food product in which a soluble dietary fiber as described herein can be used is extruded and sheeted snacks. Examples of extruded and sheeted snacks in which it can be used include puffed snacks, crackers, tortilla chips, and corn chips. In preparing an extruded piece, a soluble dietary fiber as described herein (e.g., in solid form) would be added directly with the dry products. A small amount of water would be added in the extruder, and then it would pass through various zones ranging from 100 °F to 300 °F. A soluble dietary fiber as described herein could be added at levels from 0-50% of the dry products mixture. A soluble dietary fiber as described herein in liquid form could also be added at one of the liquid ports along the extruder. The product would come out at either a low moisture content (5%) and then baked to remove the excess moisture, or at a slightly higher moisture content (10%) and then fried to remove moisture and cook out the product. Baking could be at temperatures up to 500 °F for 20 minutes. Baking would more typically be at 350 °F for 10 minutes. Frying would typically be at 350 °F for 2-5 minutes. In a sheeted snack, the resistant corn syrup solids could be used as a partial replacement of the other dry ingredients (e.g., flour). It could be from 0-50% of the dry weight. The product would be dry mixed, and then water added to form cohesive dough. The product mix could have a pH from 5 to 8. The dough would then be sheeted and cut and then baked or fried. Baking could be at temperatures up to 500 °F for 20 minutes. Frying would typically be at 350 °F for 2-5 minutes. Another potential benefit from the use of a soluble dietary fiber as described herein is a reduction of the fat content of fried snacks by as much as 15% when it is added as an internal ingredient or as a coating on the outside of a fried food.

Another type of food product in which a soluble dietary fiber as described herein can be used is gelatin desserts. The ingredients for gelatin desserts are often sold as a dry mix with gelatin as a gelling agent. The sugar solids could be replaced partially or entirely with a soluble dietary fiber as described herein in solid form in the dry mix. The dry mix can then be mixed with water and heated to 212° F. to dissolve the gelatin and then more water and/or fruit can be added to complete the gelatin dessert. The gelatin is then allowed to cool and set. Gelatin can also be sold in shelf stable packs. In that case the stabilizer is usually carrageenan-based. As stated above, a soluble dietary fiber as described herein can replace up to 100% of the other sweetener solids. The dry ingredients are mixed into the liquids and then pasteurized and put into cups and allowed to cool and set. The cups usually have a foil top.

Another type of food product in which a soluble dietary fiber as described herein can be used is cheese, cheese sauces, and other cheese products, as well as their dairy alternative versions. Examples of cheese, cheese sauces, and other cheese and dairy alternative products in which it can be used include lower milk solids cheese, lower fat cheese, and calorie reduced cheese. In block cheese, it can help to improve the melting characteristics, or to decrease the effect of the melt limitation added by other ingredients such as starch. It could also be used in cheese sauces, for example as a bulking agent, to replace fat, milk solids, or other typical bulking agents.

Another type of food product in which a soluble dietary fiber as described herein can be used is films that are edible and/or water soluble. Examples of films in which it can be used include films that are used to enclose dry mixes for a variety of foods and beverages that are intended to be dissolved in water, or films that are used to deliver color or flavors such as a spice film that is added to a food after cooking while still hot. Other film applications include, but are not limited to, fruit and vegetable leathers, and other flexible films.

Another type of food product in which a soluble dietary fiber as described herein can be used is soups, syrups, sauces, and dressings. A typical dressing could be from 0-50% oil, with a pH range of 2-7. It could be cold processed or heat processed. It would be mixed, and then stabilizer would be added. A soluble dietary fiber as described herein could easily be added in liquid or dry form with the other ingredients as needed. The dressing composition may need to be heated to activate the stabilizer. Typical heating conditions would be from 170-200° F. for 1-30 minutes. After cooling, the oil is added to make a pre-emulsion. The product is then emulsified using a homogenizer, colloid mill, or other high shear process.

Sauces can have from 0-10% oil and from 10-50% total solids, and can have a pH from 2-8. Sauces can be cold processed or heat processed. The ingredients are mixed and then heat processed. A soluble dietary fiber as described herein could easily be added in liquid or dry form with the other ingredients as needed. Typical heating would be from 170-200° F for 1-30 minutes.

Soups are more typically 20-50% solids and in a more neutral pH range (4-8). They can be a dry mix, to which a soluble dietary fiber as described herein in solid form could be added, or a liquid soup which is canned and then retorted. In soups, a soluble dietary fiber as described herein could be used up to 50% solids, though a more typical usage would be to deliver 5 g of fiber/serving.

Another type of food product in which a soluble dietary fiber as described herein can be used is coffee creamers. Examples of coffee creamers in which it can be used include both liquid and dry creamers. A dry blended coffee creamer can be blended with commercial creamer powders of the following fat types: soybean, coconut, palm, sunflower, or canola oil, or butterfat. These fats can be non-hydrogenated or hydrogenated. A soluble dietary fiber as described herein in solid form can be added as a fiber source, optionally together with fructo-oligosaccharides, polydextrose, inulin, maltodextrin, resistant starch, sucrose, and/or conventional corn syrup solids. The composition can also contain high intensity sweeteners, such as sucralose, acesulfame potassium, aspartame, or combinations thereof. These ingredients can be dry blended to produce the desired composition.

A spray dried creamer powder is a combination of fat, protein and carbohydrates, emulsifiers, emulsifying salts, sweeteners, and anti-caking agents. The fat source can be one or more of soybean, coconut, palm, sunflower, or canola oil, or butterfat. The protein can be sodium or calcium caseinates, milk proteins, whey proteins, wheat proteins, or soy proteins. The carbohydrate can be a soluble dietary fiber as described herein alone or in combination with fructo-oligosaccharides, polydextrose, inulin, resistant starch, maltodextrin, sucrose, or corn syrup. The emulsifiers can be mono- and diglycerides, acetylated mono- and diglycerides, or propylene glycol monoesters. The salts can be trisodium citrate, monosodium phosphate, disodium phosphate, trisodium phosphate, tetrasodium pyrophosphate, monopotassium phosphate, and/or dipotassium phosphate.
The composition can also contain high intensity sweeteners, such as sucralose, acesulfame potassium, aspartame, or combinations thereof. Suitable anti-caking agents include sodium silicoaluminates or silica dioxides. The products are combined in slurry, optionally homogenized, and spray dried in either a granular or agglomerated form.

Liquid coffee creamers are simply a homogenized and pasteurized emulsion of fat (either dairy fat or hydrogenated vegetable oil), some milk solids or caseinates, corn syrup, and vanilla or other flavors, as well as a stabilizing blend. The product is usually pasteurized via HTST (high temperature short time) at 185° F. for 30 seconds, or UHT (ultrahigh temperature), at 285° F. for 4 seconds, and homogenized in a two stage homogenizer at 500-3000 psi first stage, and 200-1000 psi second stage. The coffee creamer is usually stabilized so that it does not break down when added to the coffee.

Another type of food product in which a soluble dietary fiber as described herein can be used is food coatings such as icings, frostings, and glazes. In icings and frostings, a soluble dietary fiber as described herein can be used as a sweetener replacement (complete or partial) to lower caloric content and increase fiber content. Glazes are typically about 70-90% sugar, with most of the rest being water, and a soluble dietary fiber as described herein can be used to entirely or partially replace the sugar. Frosting typically contains about 2-40% of a liquid/solid fat combination, about 20-75% sweetener solids, color, flavor, and water. A soluble dietary fiber as described herein can be used to replace all or part of the sweetener solids, or as a bulking agent in lower fat systems.

Another type of food product in which a soluble dietary fiber as described herein can be used is pet food, such as dry or moist dog food. Pet foods are made in a variety of ways, such as extrusion, forming, and formulating as gravies. A soluble dietary fiber as described herein could be used at levels of 0-50% in each of these types.

Another type of food product in which a soluble dietary fiber as described herein can be used is tortillas, which usually contain flour and/or corn meal, fat, water,
salt, and fumaric acid. A soluble dietary fiber as described herein could be used to replace flour or fat. The ingredients are mixed and then sheeted or stamped and cooked. This addition could be used to add fiber or extend the shelf life.

Another type of food product in which a soluble dietary fiber as described herein can be used is fish and meat. Conventional corn syrup is already used in some meats, so a soluble dietary fiber as described herein can be used as a partial or complete substitute. For example, a soluble dietary fiber as described herein could be added to brine before it is vacuum tumbled or injected into the meat. It could be added with salt and
phosphates, and optionally with water binding ingredients such as starch, carrageenan, or soy proteins. This would be used to add fiber, a typical level would be 5 g/serving which would allow a claim of excellent source of fiber.

Another type of food product in which a soluble dietary fiber as described herein can be used is dried fish and meat snacks such as jerky. Ingredients such as conventional corn syrup, honey, sugar, agave are conventionally used in dried meat snacks to add weight. So a soluble dietary fiber as described herein can be used as a partial or complete substitute for such materials, to provide lower sugar and higher fiber to the product.

Another type of food product in which a soluble dietary fiber as described herein can be used is a meat analogue or meat alternative. Meat analogues and meat alternatives are food products used as meat substitutes and include plant-based ingredients. Meat analogs and meat alternatives can be formed without the use of animal-based ingredients, or alternatively can be made by combining animal-based ingredients with plant-based ingredients (e.g., proteins, fibers, and/or fats). Examples include texturized vegetable protein, tempeh, seitan and pea protein-based foods, as well as animal flesh analogs of the types made by Impossible Foods and Beyond Meat. Soluble dietary fiber as described above may be introduced as a modifier to any of flavor, texture and/or nutrition. For example, soluble dietary fiber may be added to texturized protein products to be used as ingredients in meat analogues, addition may be into the mass that is extruded to create the texturized protein, or after the mass has been extruded. Soluble dietary fiber may be added in a meat analogue with or without texturized protein, and it may be added pre-or post-extrusion of the meat analogue mass, or pre-or post-blending or mixing of ingredients in the composition, or pre-or post-processing. Soluble dietary fiber as described above may be homogenously dispersed throughout the product or concentrated in particular aspects of the product, for example in aspects intended to mimic animal-based components such as muscle meat, cartilage, connective and/or adipose tissue.

Another type of food product in which a soluble dietary fiber as described herein can be used is dried (infused) fruit. Many kinds of dried fruit are only stable and palatable if they are infused with sugar. A soluble dietary fiber as described herein can be substituted for all or part of the sugar. For example, a soluble dietary fiber as described herein could be added to the brine used to infuse the fruit before drying. Stabilizing agents such as sulfates can be used in this brine as well.

Another type of food product in which a soluble dietary fiber as described herein can be used is infant and toddler food. A soluble dietary fiber as described herein could be used as a replacement or a supplement to one or more conventional ingredients for such food. Because of its mild flavor and clear color, it could be added to a variety of baby foods to reduce sugar and increase fiber content.

Another type of food product in which a soluble dietary fiber as described herein can be used is batters and breadings, such as the batters and breadings for meat. This could be done by replacing all or part of the dry components of the batter and/or breading (e.g., flour type ingredients) with a soluble dietary fiber as described herein, or to use in combination with addition to the meat muscle or fried food itself. This could be used as a bulking agent, for fiber addition, or to reduce fat in the fried food.

Food products as disclosed herein can be used to help control the blood glucose concentration in mammals, such as humans, that suffer from diabetes. When the food product is consumed by the mammal, a soluble dietary fiber as described herein in the food product can cause a more moderate relative glycemic response in the bloodstream (i.e. as opposed to similar food products containing corn syrup), which can be beneficial for diabetes patients. "Control" in this context should be understood as a relative term; i.e., the glycemic response can be improved relative to that occurring when the same mammal consumes a similar food product that contains corn syrup, although the glycemic response may not necessarily be equivalent to what would be observed in a mammal that does not suffer from diabetes, or in a mammal that does not eat a food product at all.

The present inventors note that the combination of the soluble dietary fiber as described herein into food and beverage products can provide at least partial masking of otherwise undesirable flavors in such products. Without intending to be bound by theory, the inventors believe that the addition of the soluble dietary fibers described herein, with or without sweetener, can in certain embodiments help to mask harsh flavors in alcoholic beverages. Moreover, and again without intending to be bound by theory, the inventors note that use of the soluble dietary fibers described herein can provide flavor masking in foods and beverages including protein, such as protein shakes, meal replacement drinks and protein bars.

Further description is provided with respect to the Examples, below.

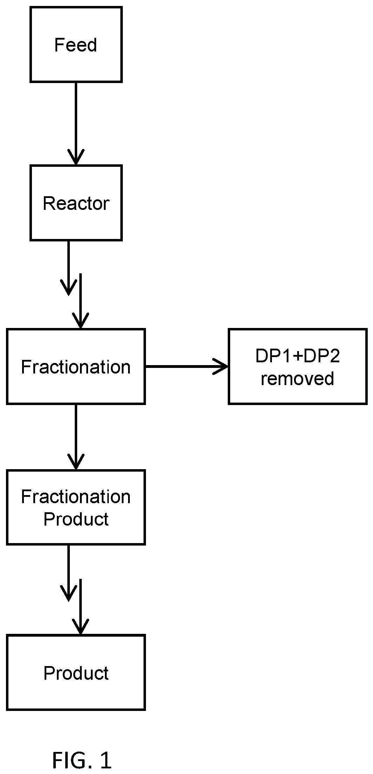

One example of a production process is shown in the flowchart of FIG. 1. Feed is reacted in a reactor, then the reaction product is fractionated. The reaction product can optionally be subjected to one or more additional process steps before fractionation, e.g., evaporation or polishing. The fractionation removes DP1/DP2 saccharides from the reaction product to provide a fractionation product, which can also be subjected to additional steps, e.g., evaporation, dilution, polishing.

### Example 1 - Preparation of reactor product composition, pilot scale

In a pilot-scale experiment, a dextrose feed comprising 99% pure dextrose was adjusted to pH 2.2-2.4 using HCl, followed by the addition of 300 ppm of H₃PO₄. The feed was then condensed to 92-96% dissolved solids in an evaporator and reacted at 440 °F (227 °C) and a 2-3 minute reactor residence time to produce a reactor product composition having a DP3+ concentration of 79.27 wt% (dry solids basis) as measured by HPLC. The DP2 content was 8.37% and the DP1 content was 8.35 wt%. The weight-average molecular weight was determined to be 1508 g/mol by gel permeation chromatography.

### Example 2 - Fractionation to provide a high-fiber product

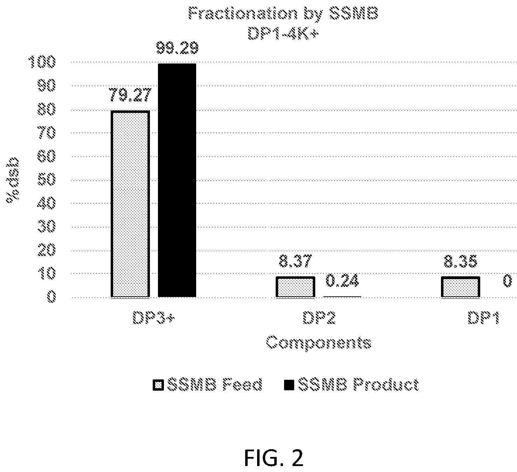

The reactor product composition from Example 1 was adjusted to 60 wt% dissolved solids and subjected to sequential simulated moving bed chromatography. The resulting high-fiber material was determined to have a DP3+ content of 99.29 wt%, a DP2 content of 0.23 wt%, a DP1 content of 0.00 wt%. FIG. 2 compares the composition of the reactor product composition and the fractionated composition. No sorbitol was detected in the product. There was about 0.48 wt% 1,6-levoglucosan. The molecular weights of the fractionated composition were determined to be 2298 g/mol (weight-average) and 1650 g/mol (number-average) by gel permeation chromatography. The fiber content as measured by AOAC 2001.03 is 105.82% on a dry solids basis. This can be compared to a first comparative commercial soluble dietary fiber product (CCSDF 1), measured to have a molecular weights of 2659 g/mol (weight-average) and 1402 g/mol (number-average), and reported to have a fiber content of 97.5% on a dry solids basis as measured by AOAC 2001.03, 994.43; and to a second comparative commercial soluble dietary fiber product (CCSDF 2), measured to have molecular weights of 3601 g/mol (weight-average) and 2090 g/mol (number average), and reported to have a fiber content (from the Certificate of Analysis; measurement technique unspecified) of 87%.

The fractionated composition had a linkage pattern comprising:
37.2% terminally-linked glucopyranosyl residues;
26.6% 6-linked glucopyranosyl residues;
7.9% 4-linked glucopyranosyl residues;
9.4% 3-linked glucopyranosyl residues;
6.6% 2-linked glucopyranosyl residues;
6.6% 4,6-linked glucopyranosyl residues;
3.8% 3,6-linked glucopyranosyl residues;
1.4% 3,4-linked glucopyranosyl residues; and
0.5% 2,4-linked glucopyranosyl residues.

Notably, the soluble dietary fiber had a ratio of 6-linked glucopyranosyl residues to 4-linked glucopyranosyl residues of about 3.37. CCSDF 1, in contrast, had a ratio of 6-linked glucopyranosyl residues to 4-linked glucopyranosyl residues of about 0.36. The ratios of alpha anomeric protons to beta anomeric protons were quantified using NMR spectroscopy. The alpha/beta ratio of the soluble dietary fiber here was 1.61. The alpha/beta ratios for CCSDF 1 and CCSDF 2 were respectively 2.9 and 3.3.

At 70% solids, the fractionated composition had a glass transition temperature as measured by differential scanning calorimetry of about -36 °C. CCSDF 1 at 70% solids had a glass transition temperature of about -38 °C. Viscosity data are provided in Table 1 below, and are reported at 10 °C, 15 °C, and 20 °C, as measured at 70% dry solids. Viscosities were measured using a stress control (DHR-3) rheometer from TA Instruments, equipped with a lower Peltier plate and an upper parallel plate (40 mm diameter). The upper parallel plate geometry was that of a previous generation of rheometer (AR-2000); in order to make it compatible with the DHR-3 rheometer, a drawdown rod and an adaptor were used. Flow curves at shear rates from 500 s-1 to 0.5 s-1 can be performed at various temperature conditions.

**Table 1**

| | Fractionated Composition of Example 2 | CCSDF 1 | CCSDF 2 |
|---|---|---|---|
| Mw (g/mol) | 2298 | 2659 | 3601 |
| Viscosity, cP (10 °C) | 41106 | 65845 | 69920 |
| Viscosity, cP (15 °C) | 25086 | 38469 | 42759 |
| Viscosity, cP (20 °C) | 15569 | 22891 | 26591 |

The fractionated composition had a water activity of about 0.893 at 70% solids, as measured by an Aqualab Series 4 from Meter Group, Inc. In contrast, CCSDF 1 had a water activity of about 0.883 at 70% solids.

### Example 3 - Fractionation to provide a high-fiber product

A reactor product composition having a DP3+ content of 80.23 wt% was fractionated to provide a high-fiber material, which was determined to have a DP3+ content of 98.36 wt%, a DP2 content of 1.48 wt%, and a DP1 content of 0.12 wt%. There was about 0.02% levoglucosans. The weight-average molecular weight of the fractionated composition was determined to be 2072 g/mol by gel permeation chromatography. The number-average molecular weight is 1372 g/mol. The fiber content as measured by AOAC 2001.03 is 104.24% on a dry solids basis.

### Example 4 - Temperature dependence of reaction

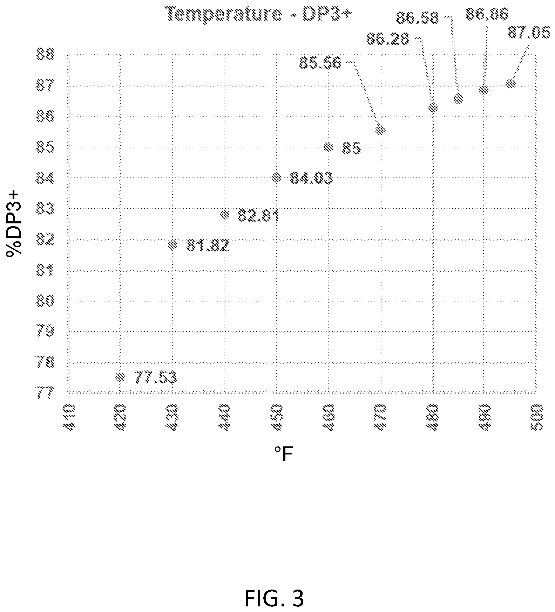

A series of reactions were performed substantially as described in Example 1, above, varying the temperature from 420-495 °F (i.e., 215-257 °C). As shown in FIG. 3, increased reaction temperatures provided increased levels of DP3+ in the reactor product composition, up to about 87% under these reaction conditions. Such higher conversions can provide higher yield and a lower cost of manufacturing, albeit at a higher molecular weight and viscosity. The person of ordinary skill in the art can select a reaction temperature and other reaction conditions to provide a desired molecular weight and rheology properties to the fiber material.

### Example 5 - Fermented Malt-type Alcoholic Beverage

A sparkling alcoholic beverage with an acidic pH can be brewed using malt and allulose. The resulting beverage has a consistent taste, mouthfeel, sweetness intensity. Exemplary compositions are shown in Table 2.

Malt extract or syrup, a concentrated, dehydrated wort resulting from crushed and mashed malted grains, can be used in place of dry malt. Liquid malt extract or syrup is typically about 25% less concentrated than dry malt.

Allulose (i.e., D-allulose, also known as D-psicose), a commercially available product that can be provided in high purity (e.g., 98% or more), can be used to provide consistent viscosity, taste, sweetness, mouthfeel, as well as mute flavor notes, alcoholic harshness, etc. like other bulk sweeteners (e.g., sucrose). In addition, allulose can modify flavors at subthreshold sweetness levels. D-allulose contained in a finally obtained fermentation solution can be provided, e.g., in amounts of 1%, 2%, or 5%.

**Table 2: Fermented Malt-type Alcoholic Beverage Recipes**

| INGREDIENT | Allulose Brew No. 1 Mass (grams) | Allulose Brew No. 2 Mass (grams) | Allulose Brew No. 3 Mass (grams) |
|---|---|---|---|
| Water | 835.99 | 825.99 | 795.99 |
| Sugar | 150 | 150 | 150 |
| Allulose | 10 | 20 | 50 |
| Stevia and/or Monk Fruit extract (optional) | 0.01 | 0.01 | 0.01 |
| Malt extract | 2 | 2 | 2 |
| Hop extract | 1.5 | 1.5 | 1.5 |
| Yeast | 0.5 | 0.5 | 0.5 |
| Flavor, acid | q.s. | q.s. | q.s. |

The components shown in Table 2 are mixed, and the resulting mixture is boiled at 80° C for 30 minutes, followed by filtration (e.g., through nylon cloth and diatomaceous earth). Yeast is added to the resulting filtrate, and fermentation by the yeast is performed at 20° C for about 10 days. Additional sugar can be added (e.g., at 5-25 g per liter of fermentation solution) to further increase specific gravity (and thus alcohol content) if desired. Fermentation is further performed at 20° C for about 14 days, whereby a sparkling malt-type alcoholic beverage is obtained.

If desired, ethanol removal and desalting can be performed according to conventional procedures. Sugar composition can be analyzed by, e.g., high-performance liquid chromatography (HPLC). The brewed beverage can be fermented until residual sugars are minimized or essentially eliminated to yield a reduced carbohydrate product that can still retain residual sweetness due to the remaining allulose (and other non-nutritive sweetener, if present).

Allulose does not hydrolyze significantly under reduced pH conditions such as those described above, nor is it significantly fermented by the yeast. Consequently, the allulose remains substantially intact, able to exert its advantageous effects on the brewed beverage as described above.

The beverages of Example 4 can optionally further include the soluble dietary fibers described herein, which can be added before, during or after fermentation. Desirable concentration ranges include between about 0.1% and 10%, or between about 0.20% and 7.5%, or between about 0.25% and 5%, or between about 0.4% and 4%, and between about 0.5% and 3%. The soluble dietary fibers described herein can improve the flavor and body profile of the alcoholic beverage, providing, e.g., an improved mouthfeel and taste experience. And in certain embodiments they can enhance sweetness or other flavors.

### Example 6 - Wine cooler-type Carbonated Alcoholic Beverage

Sparkling wine cooler-type beverages can be prepared by, e.g., combining, mixing or blending raw materials according to the exemplary compositions shown in Table 3. In addition, by using allulose in combination with the soluble dietary fibers described herein, a reduced calorie carbonated alcoholic beverage can be produced that exhibits, compared to a standard reduced calorie counterpart, a more pleasant overall taste experience characterized by a rounded, non-aggressive taste profile and full-bodied mouthfeel.

**Table 3: Exemplary Reduced Calorie Wine Cooler-type Beverage Recipes**

| INGREDIENT | Allulose/SDF Beverage No. 1 (%) | Allulose/SDF Beverage No. 2 (%) | Allulose/SDF Beverage No. 3 (%) | Allulose/SDF Beverage No. 4 (%) |
|---|---|---|---|---|
| White wine | 42 | 42 | 42 | 42 |
| Sugar (sucrose) | 6 | 6 | 6 | 6 |
| Seltzer water | 46.2 | 46.2 | 46.2 | 46.2 |
| Allulose | 2 | 2.25 | 1.75 | 1.5 |
| Stevia and/or Monk Fruit extract (optional) | 0.01 | 0.01 | 0.01 | 0.01 |
| SDF | 0.5 | 0.25 | 0.75 | 1 |
| Fruit juice | 3.0 | 3.0 | 3.0 | 3.0 |
| Citric acid | 0.1 | 0.1 | 0.1 | 0.1 |
| Fruit flavor | 0.2 | 0.2 | 0.2 | 0.2 |

Any type and varietal of wine may be used to make a wine cooler-type beverage of this example (e.g., white wines, red wines, rose wines; Chardonnay, Pinot Grigio, Sancerre, Cabernet, Merlot, etc.). The sugar source may originate from any suitable source (e.g., corn) and may be used between about 1% and 12%, or between 2% and 10%, or between about 3% and 8%, or between about 4% and 9%, or between about 5% and 8%, or between about 5% and 7%.

The SDF can be added at a concentration between about 0.1% and 5%, or between about 0.20% and 4%, or between about 0.25% and 3%, or between about 0.3% and 2%, or between about 0.4% and 2%, or between about 0.5 and 1.5%.

### Example 7 - Reduced Calorie Still Alcoholic Beverage

Reduced calorie still alcoholic beverages can be prepared by, e.g., combining, mixing or blending raw materials according to the exemplary compositions shown in Table 4.

By using the soluble dietary fibers described herein, the overall flavor profile of the still alcoholic beverage is improved and provides an improved mouthfeel and taste experience. Unpalatable notes characterized as alcoholic, aromatic and harsh are reduced delivering a higher consumer acceptance.

**Table 4: Exemplary Reduced Calorie Still Alcoholic Beverage Recipes**

| INGREDIENT | Still Alcohol No. 1 (%) | Still Alcohol No. 2 (%) | Still Alcohol No. 3 (%) | Still Alcohol No. 4 (%) |
|---|---|---|---|---|
| Water | 78 | 78.5 | 77.5 | 77 |
| Ethanol (95%) | 12 | 12 | 12 | 12 |
| Sucrose | 7.5 | 7.5 | 7.5 | 7.5 |
| SDF | 1.5 | 1.0 | 2.0 | 2.5 |
| Flavoring | 0.8 | 0.8 | 0.8 | 0.8 |
| Fumaric acid, fine | 0.2 | 0.2 | 0.2 | 0.2 |

The ethanol source can be from any fermented stock, ranging from non-distilled, yeast-fermented beverages (e.g., beers, wines, sakes) to distilled spirits, and can range from about 2.5% alcohol by volume to about 95% by volume.

The beverages of Example 6 can optionally further comprise D-allulose at a concentration between about 0.1% and 10%, or between about 0.20% and 7.5%, or between about 0.25% and 5%, or between about 0.4% and 4%, or between about 0.5% and 3%. D-allulose helps provide consistent viscosity, taste, sweetness, satisfying mouthfeel, and also mutes harsh alcoholic flavor notes. Allulose can be added in addition to a specified amount of sucrose or can replace sucrose in coincident amounts.

### Example 8 - Fruit-based Beverage/Liqueur with allulose and Soluble Dietary Fiber

Flavored alcohol-containing beverages (e.g., wines, spirits, liquors, etc.) can be prepared by , e.g., combining, mixing or blending raw materials according to the composition shown in the following Table 5. The beverage may be matured over a desired period of time at one or more preferred temperatures and in one or more preferred storage vessels (e.g., stainless steel, glass, wood barrels and the like) to allow for infusion of flavors from the fruit component (and from the storage vessel in the case of storage in wood) into the final beverage.

**Table 5: Exemplary Fruit-based Beverage/Liqueur with allulose and SDF Recipes**

| INGREDIENT | Beverage No. 1 Mass (grams) | Beverage No. 2 Mass (grams) | Beverage No. 3 Mass (grams) | Beverage No. 4 Mass (grams) |
|---|---|---|---|---|
| Fruit puree (with solids) | 1000 | 1000 | 1000 | 1000 |
| Ethanol source | 1800 | 1800 | 1800 | 1800 |
| Allulose | 750 | 1000 | 1250 | 1250 |
| Stevia and/or Monk Fruit extract (optional) | 0.01-0.1 | 0.01-0.1 | 0.01-0.1 | 0.01-0.1 |
| Soluble dietary fiber (SDF) | 54 | 57 | 61 | 61 |

The ethanol source can be from any fermented stock, ranging from non-distilled, yeast-fermented beverages (e.g., beers, wines, sakes) to distilled spirits, and can range from about 2.5% alcohol by volume to about 95% by volume.

By using the soluble dietary fiber as described herein, the overall flavor profile of the still alcoholic beverage is improved, providing, e.g., an improved mouthfeel and taste experience. Unpalatable notes characterized as alcoholic, aromatic and harsh are reduced providing a higher consumer satisfaction.

## Claims

1. A soluble dietary fiber having a fiber content of at least 97% as measured by AOAC 2001.03 and a combined DP1 + DP2 content of no more than 3 wt% on a dry solids basis, and a linkage pattern comprising:
30-45% terminally-linked glucopyranosyl residues;
18-30% 6-linked glucopyranosyl residues;
4-12% 4-linked glucopyranosyl residues;
4-13% 3-linked glucopyranosyl residues;
3-8% 2-linked glucopyranosyl residues;
2-10% 4,6-linked glucopyranosyl residues;
2-7% 3,6-linked glucopyranosyl residues;
up to 4% 3,4-linked glucopyranosyl residues; and
up to 4% 2,4-linked glucopyranosyl residues.

2. A soluble dietary fiber according to claim 1, having a fiber content of 97% to 108%, e.g., 97% to 106%, or 97% to 103%, or 97% to 100%.

3. A soluble dietary fiber according to claim 1 or claim 2, having a DP1 + DP2 content of no more than 2 wt% on a dry solids basis, e.g., no more than 1.7 wt%.

4. A soluble dietary fiber according to any of claims 1-3, having a DP2 content of no more than 1.0 wt% on a dry solids basis, e.g., no more than 0.75 wt% and/or having a DP1 content of no more than 0.50 wt% on a dry solids basis, e.g., no more than 0.30 wt%, or no more than 0.20 wt%.

5. A soluble dietary fiber according to any of claims 1-4, having at least 99 wt% dextrose residues (e.g., at least 99.5 wt%, or at least 99.8 wt% dextrose residues) on a dry solids basis and/or having no more than 0.2 wt% sugar alcohol residues on a dry solids basis, e.g., no more than 0.1 wt% sugar alcohol residues.

6. A soluble dietary fiber according to any of claims 1-5, wherein the soluble dietary fiber has a weight-average molecular weight in the range of 1000 g/mol to 2500 g/mol and/or a polydispersity of no more than 1.8, e.g., no more than 1.7, or no more than 1.6.

7. A soluble dietary fiber according to any of claims 1-6, having a linkage pattern comprising:
35-43% terminally-linked glucopyranosyl residues;
20-28% 6-linked glucopyranosyl residues;
6-11% 4-linked glucopyranosyl residues;
6-12% 3-linked glucopyranosyl residues;
3-8% 2-linked glucopyranosyl residues;
3-9% 4,6-linked glucopyranosyl residues;
2-7% 3,6-linked glucopyranosyl residues;
up to 3% 3,4-linked glucopyranosyl residues; and
up to 2% 2,4-linked glucopyranosyl residues.

8. A soluble dietary fiber according to any of claims 1-7, having a ratio of 6-linked glucopyranosyl residues to 4-linked glucopyranosyl residues that is at least 2, e.g., at least 2.5 and/or a ratio of alpha anomeric protons to beta anomeric protons in the range of 1.2-2.

9. A soluble dietary fiber according to any of claims 1-8, having a glass transition temperature at 70% solids in the range of -20 °C to -50 °C and/or having a viscosity at 70% DS and 10 °C of no more than 55000 cP, for example, no more than 50000 cP, or no more than 45000 cP.

10. A method of making a soluble dietary fiber according to any of claims 1-9, comprising providing a saccharide feed comprising at least 95 wt% (e.g., at least 97 wt%, at least 98 wt% or at least 99 wt%) on a dry solids basis of dextrose and/or dextrose oligomers (e.g., linear dextrose oligomers);
reacting the saccharide feed in the presence of water and in the substantial absence of sugar alcohols at a total solids concentration of at least 80% by weight and a temperature of at least 120 °C with at least one acid catalyst that accelerates the rate of cleavage and formation of glucosyl bonds for a time sufficient to produce a reactor product composition having a DP3+ of at least 60 wt% on a dry solids basis; and
fractionating the reactor product composition to separate DP1 and DP2 and to provide a high-fiber fraction having a fiber content of at least 97% as measured by AOAC 2001.03.

11. A method of making a soluble dietary fiber according to claim 10, comprising fractionating the reactor product composition to separate DP1 and DP2 and to provide a
high-fiber fraction having a DP1+DP2 value of no more than 3 wt% (e.g., no more than 2 wt%).

12. A method for improving body and mouthfeel in a fermented beverage, the method including providing a soluble dietary fiber of any of claims 1-9 in the fermented beverage.

13. A method for making a food or beverage product, the method comprising:
providing a soluble dietary fiber according to any of claims 1-9 and
combining the soluble dietary fiber with one or more other food or beverage ingredients.

14. A food or beverage product comprising the soluble dietary fiber according to any of claims 1-9.

15. A beverage product according to claim 14, wherein the beverage product is a fermented beverage.

## Patentansprüche

1. Löslicher Ballaststoff, der einen Ballaststoffgehalt von mindestens 97 %, gemessen gemäß AOAC 2001.03, und einen kombinierten DP1 + DP2-Gehalt von höchstens 3 Gew.- % bezogen auf eine Trockenmasse und ein Verknüpfungsmuster aufweist, das Folgendes umfasst:
30-45 % endständig verknüpfte Glucopyranosylreste;
18-30 % 6-verknüpfte Glucopyranosylreste;
4-12 % 4-verknüpfte Glucopyranosylreste;
4-13 % 3-verknüpfte Glucopyranosylreste;
3-8 % 2-verknüpfte Glucopyranosylreste;
2-10 % 4,6-verknüpfte Glucopyranosylreste;
2-7 % 3,6-verknüpfte Glucopyranosylreste;
bis zu 4 % 3,4-verknüpfte Glucopyranosylreste; und
bis zu 4 % 2,4-verknüpfte Glucopyranosylreste.

2. Löslicher Ballaststoff nach Anspruch 1, der einen Ballaststoffgehalt von 97 % bis 108 %, z. B. 97 % bis 106 % oder 97 % bis 103 % oder 97 % bis 100 % aufweist.

3. Löslicher Ballaststoff nach Anspruch 1 oder 2, der einen DP1 + DP2-Gehalt von höchstens 2 Gew.-% bezogen auf eine Trockenmasse, z. B. höchstens 1,7 Gew.-% aufweist.

4. Löslicher Ballaststoff nach einem der Ansprüche 1-3, der einen DP2-Gehalt von höchstens 1,0 Gew.-% bezogen auf eine Trockenmasse, z. B. höchstens 0,75 Gew.-%. aufweist und/oder einen DP1-Gehalt von höchstens 0,50 Gew.-% bezogen auf eine Trockenmasse, z. B. höchstens 0,30 Gew.-% oder höchstens 0,20 Gew.-% aufweist.

5. Löslicher Ballaststoff nach einem der Ansprüche 1-4, der mindestens 99 Gew.-% Dextrosereste (z. B. mindestens 99,5 Gew.-% oder mindestens 99,8 Gew.-% Dextrosereste) bezogen auf eine Trockenmasse aufweist und/oder nicht mehr als 0,2 Gew.-% Zuckeralkoholrückstände bezogen auf eine Trockenmasse, z.B. nicht mehr als 0,1 Gew.-% Zuckeralkoholrückstände aufweist.

6. Löslicher Ballaststoff nach einem der Ansprüche 1 bis 5, wobei der lösliche Ballaststoff ein durchschnittliches Molekulargewicht im Bereich von 1000 g/mol bis 2500 g/mol und/oder eine Polydispersität von nicht mehr als 1,8, z. B. höchstens 1,7 oder höchstens 1,6 aufweist.

7. Löslicher Ballaststoff nach einem der Ansprüche 1 bis 6,der ein Verknüpfungsmuster aufweist, das Folgendes umfasst:
35-43 % endständig verknüpfte Glucopyranosylreste;
20-28 % 6-verknüpfte Glucopyranosylreste;
6-11 % 4-verknüpfte Glucopyranosylreste;
6-12 % 3-verknüpfte Glucopyranosylreste;
3-8 % 2-verknüpfte Glucopyranosylreste;
3-9 % 4,6-verknüpfte Glucopyranosylreste;
2-7 % 3,6-verknüpfte Glucopyranosylreste;
bis zu 3 % 3,4-verknüpfte Glucopyranosylreste; und
bis zu 2 % 2,4-verknüpfte Glucopyranosylreste.

8. Löslicher Ballaststoff nach einem der Ansprüche 1-7, der ein Verhältnis von 6-verknüpften Glucopyranosylresten zu 4-verknüpften Glucopyranosylresten von mindestens 2, z. B. mindestens 2,5 und/oder ein Verhältnis von alpha-anomeren Protonen zu beta-anomeren Protonen im Bereich von 1,2-2 aufweist.

9. Löslicher Ballaststoff nach einem der Ansprüche 1-8, der eine Glasübergangstemperatur bei 70 % Feststoff im Bereich von -20 °C bis -50 °C und/oder eine Viskosität bei 70 % Trockenmasse und 10 °C von höchstens 55.000 cP, beispielsweise höchstens 50.000 cP oder höchstens 45.000 cP aufweist.

10. Verfahren zur Herstellung eines löslichen Ballaststoffs nach einem der Ansprüche 1 bis 9, umfassend
Bereitstellen einer Saccharid-Zufuhr, die mindestens 95 Gew.-% (z. B. mindestens 97 Gew.-%, mindestens 98 Gew.-% oder mindestens 99 Gew.-%) bezogen auf eine Trockenmassen von Dextrose und/oder Dextrose-Oligomeren (z. B. lineare Dextrose-Oligomere) umfasst;
Umsetzen der Saccharid-Zufuhr in Gegenwart von Wasser und in wesentlicher Abwesenheit von Zuckeralkoholen bei einer Gesamtfestmassekonzentration von mindestens 80 Gew.-% und einer Temperatur von mindestens 120 °C mit mindestens einem Säurekatalysator, der die Spaltungs- und Bildungsgeschwindigkeit von Glucosylbindungen für eine ausreichende Zeit beschleunigt, um eine Reaktorproduktzusammensetzung zu erzeugen, die einen DP3+ von mindestens 60 Gew.- % bezogen auf eine Trockenmasse aufweist; und
Fraktionieren der Reaktorproduktzusammensetzung zum Abscheiden von DP1 und DP2 und Bereitstellen einer faserreichen Fraktion, die einen Ballaststoffgehalt von mindestens 97 %, gemessen gemäß AOAC 2001.03, aufweist.

11. Verfahren zur Herstellung eines löslichen Ballaststoffs nach Anspruch 10, umfassend
Fraktionieren der Reaktorproduktzusammensetzung zum Abscheiden von DP1 und DP2 und Bereitstellen einer faserreichen Fraktion, die einen DP1+DP2-Wert von höchstens 3 Gew.-% (z.B. nicht mehr als 2 Gew.-%) aufweist.

12. Verfahren zur Verbesserung von Körper und Mundgefühl in einem fermentierten Getränk, wobei das Verfahren einen löslichen Ballaststoff nach einem der Ansprüche 1-9 in dem fermentierten Getränk bereitstellt.

13. Verfahren zur Herstellung eines Lebensmittels oder Getränks, wobei das Verfahren Folgendes umfasst:
Bereitstellen eines löslichen Ballaststoffs nach einem der Ansprüche 1-9 und
Kombinieren des löslichen Ballaststoffs mit einem oder mehreren anderen Lebensmittel- oder Getränkebestandteilen.

14. Lebensmittel oder Getränke, das den löslichen Ballaststoff nach einem der Ansprüche 1-9 umfasst.

15. Getränkeerzeugnis nach Anspruch 14, wobei das Getränkeerzeugnis ein fermentiertes Getränk ist.

## Revendications

1. Fibre alimentaire soluble présentant une teneur en fibres d'au moins 97 % telle que mesurée par AOAC 2001.03 et une teneur combinée en DP1 + DP2 ne dépassant pas 3 % en poids sur une base de matières solides sèches, et un motif de liaison comprenant :
30-45 % de résidus glucopyranosyles liés en position terminale ;
18-30 % de résidus glucopyranosyles liés en position 6 ;
4-12 % de résidus glucopyranosyles liés en position 4 ;
4-13 % de résidus glucopyranosyles liés en position 3 ;
3-8 % de résidus glucopyranosyles liés en position 2 ;
2-10 % de résidus glucopyranosyles liés en position 4,6 ;
2-7 % de résidus glucopyranosyles liés en position 3,6 ;
jusqu'à 4% de résidus glucopyranosyles liés en position 3,4 ; et
jusqu'à 4% de résidus glucopyranosyles liés en position 2,4.

2. Fibre alimentaire soluble selon la revendication 1, présentant une teneur en fibres de 97 % à 108 %, par exemple, de 97 % à 106 %, ou de 97 % à 103 %, ou de 97 % à 100 %.

3. Fibre alimentaire soluble selon la revendication 1 ou la revendication 2, présentant une teneur en DP1 + DP2 ne dépassant pas 2 % en poids sur une base de matières solides sèches, par exemple, ne dépassant pas plus de 1,7 % en poids.

4. Fibre alimentaire soluble selon l'une quelconque des revendications 1-3, présentant une teneur en DP2 ne dépassant pas 1,0 % en poids sur une base de matières solides sèches, par exemple, ne dépassant pas 0,75 % en poids et/ou présentant une teneur en DP1 ne dépassant pas 0,50 % en poids sur une base de matières solides sèches, par exemple, ne dépassant pas 0,30 % en poids, ou ne dépassant pas 0,20 % en poids.

5. Fibre alimentaire soluble selon l'une quelconque des revendications 1-4, présentant au moins 99 % en poids de résidus de dextrose (par exemple, au moins 99,5 % en poids, ou au moins 99,8 % en poids de résidus de dextrose) sur une base de matières solides sèches et/ou ne présentant pas plus de 0,2 % en poids de résidus d'alcool de sucre sur une base de matières solides sèches, par exemple pas plus de 0,1 % en poids de résidus d'alcool de sucre.

6. Fibre alimentaire soluble selon l'une quelconque des revendications 1-5, dans laquelle la fibre alimentaire soluble présente un poids moléculaire moyen de l'ordre de 1000 g/mol jusqu'à 2500 g/mol et/ou une polydispersité ne dépassant pas 1,8, par exemple ne dépassant pas 1,7 ou ne dépassant pas 1,6.

7. Fibre alimentaire soluble selon l'une quelconque des revendications 1-6, présentant un motif de liaison comprenant :
35-43 % de résidus glucopyranosyles liés en position terminale ;
20-28 % de résidus glucopyranosyles liés en position 6 ;
6-11 % de résidus glucopyranosyles liés en position 4 ;
6-12 % de résidus glucopyranosyles liés en position 3 ;
3-8 % de résidus glucopyranosyles liés en position 2 ;
3-9 % de résidus glucopyranosyles liés en position 4,6 ;
2-7 % de résidus glucopyranosyles liés en position 3,6 ;
jusqu'à 3 % de résidus glucopyranosyles liés en position 3,4 ; et
jusqu'à 2 % de résidus glucopyranosyles liés en position 2,4.

8. Fibre alimentaire soluble selon l'une quelconque des revendications 1-7, présentant un rapport entre les résidus de glucopyranosyle liés en position 6 et les résidus de glucopyranosyle liés en position 4, qui est au moins de 2, par exemple au moins de 2,5 et/ou un rapport entre les protons anomériques alpha et les protons anomériques bêta dans la plage de 1,2-2.

9. Fibre alimentaire soluble selon l'une quelconque des revendications 1-8, présentant une température de transition vitreuse à 70 % de matières solides comprise dans la plage entre -20 °C et -50 °C et/ou présentant une viscosité à 70 % DS et à 10 °C ne dépassant pas 55 000 cP, par exemple ne dépassant pas 50 000 cP ou ne dépassant pas 45 000 cP.

10. Procédé de fabrication d'une fibre alimentaire soluble selon l'une quelconque des revendications 1-9, comprenant
la fourniture d'une alimentation en saccharides comprenant au moins 95 % en poids (par exemple, au moins 97 % en poids, au moins 98 % en poids ou au moins 99 % en poids) sur une base de matières solides sèches de dextrose et/ou d'oligomères de dextrose (par exemple, oligomères de dextrose linéaires) ;
la réaction de l'alimentation en saccharides en présence d'eau et en l'absence substantielle d'alcools de sucre à une concentration totale en matières solides d'au moins 80 % en poids et à une température d'au moins 120 °C avec au moins un catalyseur acide qui accélère la vitesse de clivage et de formation des liaisons glucosyliques pendant une durée suffisante pour produire une composition de produit de réacteur présentant un DP3+ d'au moins 60 % en poids sur une base de matières solides sèches ; et
le fractionnement de la composition de produit de réacteur pour séparer DP1 et DP2 et fournir une fraction à haute teneur en fibres présentant une teneur en fibres d'au moins 97 % telle que mesurée par AOAC 2001.03.

11. Procédé de fabrication d'une fibre alimentaire soluble selon la revendication 10, comprenant
le fractionnement de la composition de produit de réacteur pour séparer DP1 et DP2 et fournir une fraction à haute teneur en fibres présentant une valeur DP1+DP2 ne dépassant pas 3 % en poids (par exemple, ne dépassant pas 2 % en poids).

12. Procédé pour améliorer la texture et la sensation en bouche d'une boisson fermentée, le procédé incluant l'apport d'une fibre alimentaire soluble selon l'une quelconque des revendications 1-9 dans la boisson fermentée.

13. Procédé de fabrication d'un produit alimentaire ou à boire, le procédé comprenant :
la fourniture d'une fibre alimentaire soluble selon l'une quelconque des revendications 1-9 et
l'association de la fibre alimentaire soluble à un ou plusieurs autres ingrédients alimentaires ou à boire.

14. Produit alimentaire ou à boire comprenant la fibre alimentaire soluble selon l'une quelconque des revendications 1-9.

15. Produit à boire selon la revendication 14, dans lequel le produit à boire est une boisson fermentée.
